(19) Europäisches Patentamt
European Patent Office
Office européen des brevets

(11) Veröffentlichungsnummer: **0 109 020**
**B1**

(12) # EUROPÄISCHE PATENTSCHRIFT

(45) Veröffentlichungstag der Patentschrift: **23.09.87**

(51) Int. Cl.⁴: **C 07 K 5/06**, C 07 D 209/58, A 61 K 37/02

(21) Anmeldenummer: **83111079.6**

(22) Anmeldetag: **07.11.83**

(54) **Neue Derivate tricyclischer Aminosäuren, Verfahren zu ihrer Herstellung, diese enthaltende Mittel und deren Verwendung, sowie neue bicyclische Aminosäuren als Zwischenstufen und Verfahren zu deren Herstellung.**

(30) Priorität: **13.11.82 DE 3242151**

(43) Veröffentlichungstag der Anmeldung: **23.05.84 Patentblatt 84/21**

(45) Bekanntmachung des Hinweises auf die Patenterteilung: **23.09.87 Patentblatt 87/39**

(84) Benannte Vertragsstaaten: **AT BE CH DE FR GB IT LI LU NL SE**

(56) Entgegenhaltungen: **EP-A-0 088 350**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Völklinger Weg 56, D-6000 Frankfurt am Main 71 (DE)**
Erfinder: **Becker, Reinhard, Dr., Adelheidstrasse 101, D-6200 Wiesbaden (DE)**

**EP 0 109 020 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Derivate der tricyclischen Aminosäuren der Formel

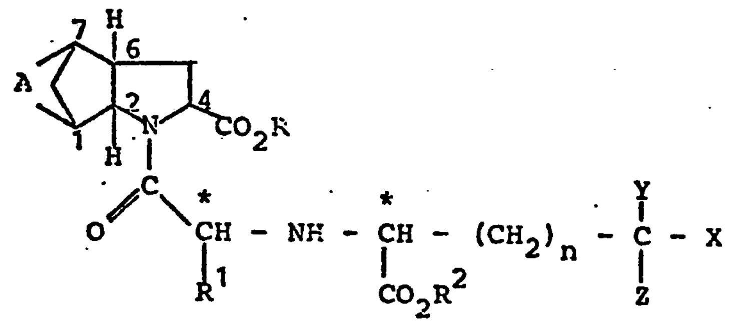

(I)

in der
n = 0 oder 1,
A = -CH = CH- oder $-CH_2 - CH_2$
R = Wasserstoff, ($C_1$ bis $C_6$)-Alkyl odor Aralkyl mit 7 bis 9 C-Atomen,
$R^1$ = Wasserstoff oder ($C_1$ bis $C_6$)-Alkyl, das gegebenenfalls durch Amino ($C_1$ bis $C_4$)-Acylamino, insbesondere Alkanoylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogon substituiert sein kann, ($C_6$ bis $C_{12}$)-Aryl-($C_1$ bis $C_4$)-alkyl oder ($C_7$ bis $C_{13}$)-Aroyl-($C_1$ bis $C_4$)-alkyl die beide wie vorstsehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer gegebenfalls geschützten natürlich vorkommenden Aminosäure,
$R^2$ = Wasserstoff, ($C_1$ bis $C_6$)-Alkyl, ($C_2$ bis $C_6$)-Alkenyl oder ($C_6$-$C_{12}$)-Aryl-($C_1$ bis $C_4$)-alkyl
Y = Wasserstoff oder Hydroxy,
Z = Wasserstoff oder
Y und Z = zusammen Sauerstoff und
X = ($C_1$ bis $C_6$)-Alkyl, ($C_2$ bis $C_6$)-Alkenyl, ($C_5$ bis $C_9$)-Cycloalkyl, ($C_6$-$C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkyl-amino und/oder Methylendioxy mono-, di- oder trisubstituiert sein kann, oder 3-Indolyl bedeuten,
sowie deren physiologisch unbedenkliche Salze.

Falls $R^1$ für eine Seitenkette einer geschützten natürlich vorkommenden α-Aminosäure steht, wie z. B. geschütztes Ser, Thr, Asp, Asn, Glu, Gln, Arg, Lys, Hyl, Cys, Orn, Cit, Tyr, TrP, His oder Hyp, sind als Schutzgruppen die in der Peptidchemie üblichen Gruppen bevorzugt (vgl. Houben-Weyl, Bd. XV/1 und XV/2)). Im Falle, daß $R^1$ die geschützten Lysin-Seitenkette bedeutet, werden die bekannten Amino-Schutzgruppen, insbesondore aber ($C_1$-$C_6$)-Alkanoyl bevorzugt. Als O-Schutzgruppen für Tyrosin kommen bevorzugt Methyl und Ethyl in Frage.

Als Salze kommen insbesondere Alkali- und Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Unter Aryl ist hier wie im folgenden vorzugsweise gegebenenfalls substituiertes Phenyl oder Naphthyl zu verstehen. Entsprechendes gilt für die Aroylreste. Alkyl kann geradkettig oder verzweigt sein.

Die Konfiguration der H-Atome an C-2 und C-6 der Tricyclen ist durch die cis Konfiguration bestimmt. Weiterhin kommen zwei mögliche Konfigurationen der H-Atome an C-2 und C-6 des Tricyclus in Betracht und zwar die exo-Stellung der H-Atome bezüglich des bicyclischen [2.2.1] Ringteils (Formelrest Ia) und entsprechend die endo-Stellung (Formelrest Ib).

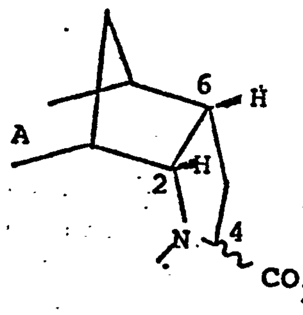
(Ia)

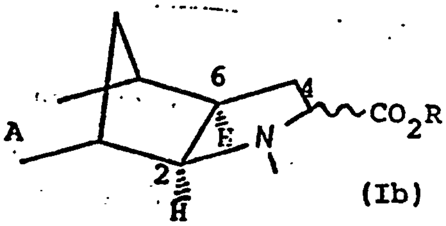
(Ib)

Die Carboxylgruppe an C-Atom 4 kann sowohl im Formelrest Ia als auch im Formelrest Ib trans (Formelrest Ic + If) oder cis-ständig (Formelrest Id + Ie) zum Wasserstoff an C-Atom 2 orientiert sein. Alle oben erwähnten Konfigurationsisomere sowie die Spiegelbildisomeren der Formeln Ic bis If sind Gegenstand dieser Erfindung.

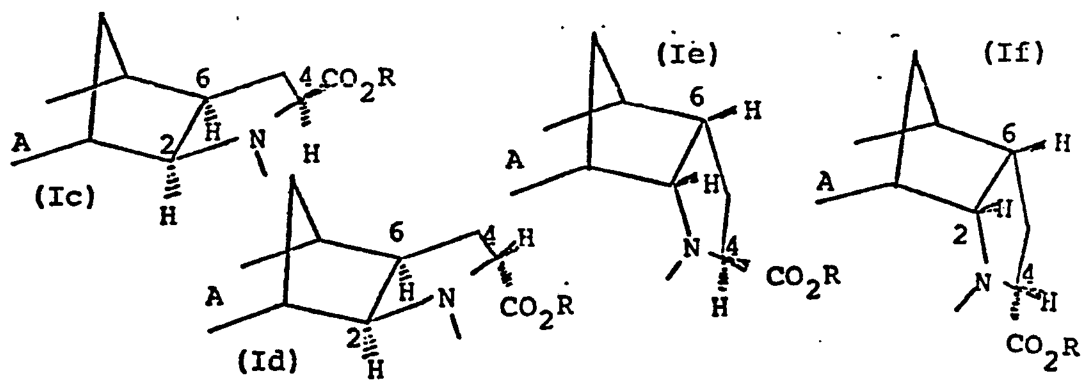
(Ic) (Id) (Ie) (If)

Verbindungen der Formel I besitzen chirale C-Atome in den Positionen C-1, C-2, C-4, C-6, C-7 sowie in den mit einem Stern markierten C-Atomen der Seitenkette. Sowohl die R- als auch die S-Konfigurationen an allen Zentren sind Gegenstand dei Erfindung. Die Verbindungen der Formel I können daher als optische Isomere, als Diastereomere, als Racemate oder als Gemische derselben vorliegen. Bevorzugt sind jedoch die Verbindungen der Formel I, in denen das C-Atom 4 im tricyclischen Ringsystem, sowie die mit einem Stern (*) markierten C-Atome der Seitenkette S-Konfiguration aufweisen, mit der Ausnahme von (-CO-*CHR$^1$-NH-) = Cys, wo die R-Konfiguration bevorzugt ist.

Besonders bevorzugte Verbindungen der Formel 1 sind solche, in denen
n = 1,
A = CH=CH oder $CH_2$-$CH_2$
R = Wasserstoff oder Alkyl mit 1 bis 4 C-Atomen,
R$^1$ = Wasserstoff, ($C_1$ bis $C_3$)-Alkyl, ($C_2$ oder $C_3$)-Alkenyl, Benzyl, 4-Alkoxybenzyl, Phenethyl, 4-Amino-butyl oder Benzoylmethyl,
R$^2$ = Wasserstoff, ($C_1$ bis $C_4$)-Alkyl oder Benzyl und
X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkyl-amino, Nitro und/oder Methylendioxy mono- oder disubstituiert oder im Falle von Methoxy trisubstituiert sein kann, bedeuten,
insbesondere solche Verbindungen der Formel I, in denen n = 1, A = $CH_2$-$CH_2$, R = Wasserstoff, R$^1$ = Methyl oder der Rest einer gegebenenfalls geschützten natürlichen Aminosäure, X = Phenyl,
R$^2$ = Wasserstoff oder Ethyl bedeuten, die Wasserstoff-Atome an C-2 und C-6 die cis-Konfiguration und exo- oder endo-Konfiguration bezüglich des bicyclischen [2.2.1]-Gerüstes besitzen, die-Carboxylgruppe an C-4 cis oder trans zum Wasserstoff an C-2 orientiert ist und die chiralen C-Atome, die mit einem Stern (*) gekennzeichnet sind und C-Atom 4 die S-Konfiguration besitzen.

Ganz besonders bevorzugt sind die Verbindungen endo-exo: N-(1-S-Carbethoxy-3-phenyl-propyl)-S-alanyl-1R,2R,4S,6S,7S-tricyclo[5.2.1.0$^{2-6}$]-3-aza-decan-4-carbonsäure
N-(1-S-Carboxy-3-phenyl-propyl)-S-alanyl-1R,2R,4S,6S,7S-tricyclo [5.2.1.0$^{2.6}$]-3-aza-decan-4-carbonsäure
N$_\alpha$-(1-S-Carbethoxy-3-phenyl-propyl)-S-lysyl-1R,2R,4S,6S,7S-tricyclo[5.2.1.0$^{2.6}$]-3-aza-decan-4-carbonsäure
N$_\alpha$-(1-S-Carboxy-3-phenyl-propyl)-S-lysyt-1R,2R,4S,6S,7S-tricyclo[5.2.1.0$^{2.6}$]-3-aza-decan-4-carbonsäure
endo-endo
-N-(1-S-Carbethoxy-3-phenyl-propyl)-S-alanyl-1S,2S,4S,6R,7R-tricyclo[5.2.1.0$^{2.6}$]-3-aza-decan-4-carbonsäure
N-(1-S-Carboxy-3-phenyl-propyl)-3-alanyl-1S,2S,4S,6R,7R-tricyclo[5.2.1.0$^{2.6}$]-3-aza-decan-4-carbonsäure
N$_\alpha$-(1-S-Carbethoxy-3-phenyl-propyl)-S-lysyl-1S 2S,4S,6R,7R -tricyclo[5.2.1.0$^{2.6}$]-3-aza-decan-4-carbonsäure
N$_\alpha$-(1-S-Carboxy-3-phenyl-propyl)-S-lysyl-1S,2S,4S,6R,7R-tricyclo[5.2.1.0$^{2.6}$]-3-aza-decan-4-carbonsäure

exo-endo
N-(1-S-Carbethoxy-3-phenyl-propyl)-S-alanyl-1R,2S,4S,6R,7S-tricyclo[5.2.1.$0^{2.6}$]-3-aza-decan-4-carbonsäure
N-(1-S-Carboxy-3-phenyl-propyl)-S-alanyl-1R,2S,4S,6R,7S -tricyclo[5.2.1.$0^{2.6}$]-3-aza-decan-4-carbonsäure
$N_\alpha$-(1-S-Carbethoxy-3-phenyl-propyl)-S-lysyl-1R, 2S, 4S, 6R, 7S-tricyclo[5.2.1.$0^{2.6}$]-3-aza-decan-4-carbonsäure
$N_\alpha$-(1-S-Carboxy-3-phenyl-propyl)-S-lysyl-1R,2S,4S,6R,7S-tricyclo[5.2.1.$0^{2.6}$]-3-aza-decan-4-carbonsärue
exo-exo
N-(1-S-Carbethoxy-3-phenyl-propyl)-S-alanyl-1S,2R,4S,6S,7R-tricyclo[5.2.1.$0^{2.6}$]-3-aza-decan-4-carbonsäure
N-(1-S-Carboxy-3-phenyl-propyl)-S-alanyl-1S,2R,4S,6S,7R-tricyclo[5.2.1.$0^{2.6}$]-3-aza-decan-4-carbonsäure
$N_\alpha$-(1-S-Carbethoxy-3-phenyl-propyl)-S-lysyl-1S,2R,4S,6S,7R-tricyclo[5.2.1.$0^{2.6}$]-3-aza-decan-4-carbonsäure
$N_\alpha$-(1-S-Carboxy-3-phenyl-propyl)-S-lysyl-1S,2R,4S,6S,7R-tricyclo[5.2.1.$0^{2.6}$]-3-aza-decan-4-carbonsäure

Die Erfindung betrifft ferner Verfahren zur Herstellung der Verbindungen der Formel I. Eine Verfahrensvariante ist dadurch gekennzeichnet, daß man eine Verbindung der Formel II,

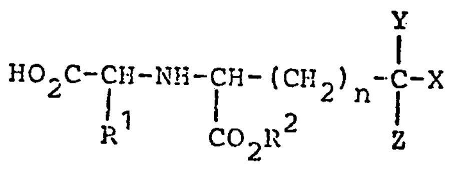

(II)

worin n, $R^1$, $R^2$, X, Y und Z die Bedeutungen wie in Formel I haben, mit einer Verbindung der Formel III,

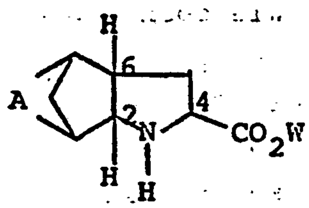

(III)

in welcher
A die Bedeutung wie in Formel I hat und
W = Wasserstoff oder einensauer, basisch oder hydrogenolytisch abspaltbaren Rest, insbesondere einen tert.-Butyl- oder Benzyl-Rest bedeuten,
nach bekannten Amidbildungsmethoden der Peptidchemie umsetzt und gegebenenfalls anschließend durch Säure- oder Basenbehandlung oder Hydrogenolyse den Rest W und gegebenenfalls durch zusätzliche Säure- oder Basenbehandlung auch den Rest $R^2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV,

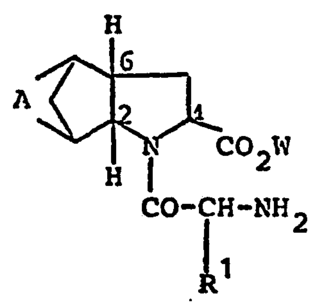

(IV)

in welcher $R^1$ und A die Bedeutung wie in Formel I und W die Bedeutung wie in Formel III besitzen, mit einer Verbindung der Formel V

$$R^2O_2C\text{-}CH=CH\text{-}CO\text{-}X \qquad (V)$$

worin $R^2$ und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest $R^2$, wie oben beschrieben, abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$OHC-CO_2R^2$

(VI)

$X-CO-CH_3$

(VII)

worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest $R_2$ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem Soc. 93, 2897 (1971) beschriebenen Verfahrensweise mit einer Verbindung der Formel VIII

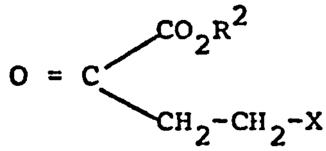

(VIII)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann katalytisch, elektrolytisch oder mit Reduktionsmitteln wie beispielsweise komplexen Boranaten, vorzugsweise Natriumborhydrid oder Natriumcyanborhydrid erfolgen.

Verbindungen der Formel 1 mit Y = Hydroxy und Z = Wasserstoff können beispielsweise auch durch Reduktion einer gemäß obigen Verfahrensweisen erhaltenen Verbindung mit Y und Z = zusammen Sauerstoff erhalten werden. Diese Reduktion kann katalytisch mit Wasserstoff oder mit einem anderen Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$-$C_9$)-Aralkyl bedeutet, überführt werden.

Die Erfindung betrifft auch Verbindungen der Formel III, in der die H-Atome an den C-Atomen 2 und 6 zueinander cis-konfiguiert sind, der Pyrrolidinring endo- oder exoständig zum bicyclischen Ringsystem orientiert ist und die Gruppe $-CO_2W$ an C-Atom 4 cis- oder trans-ständig zum Wasserstoff an C-Atom 2 orientiert ist und worin W = Wasserstoff oder einen sauer, basisch oder hydrogenolytisch abspaltbaren Rest und A eine CH=CH oder $CH_2$-$CH_2$-Gruppe bedeutet.

Diese Verbindungen dienen gemäß der Erfindung als Ausgangsstoffe bei der Synthese von Verbindungen der Formel I und können erfindungsgemäß nach folgender Verfahrensweise hergestellt werden:

Bei einer Synthesevariante geht man von einer Verbindung der Formel IX oder X aus,

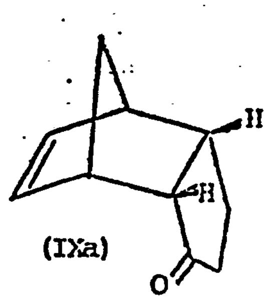

(IXa)

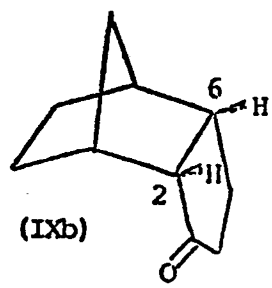

(IXb)

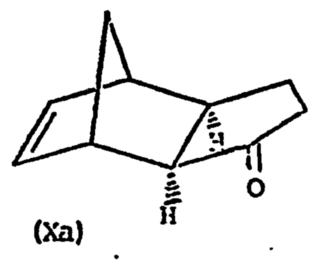

(Xa)

(Xb)

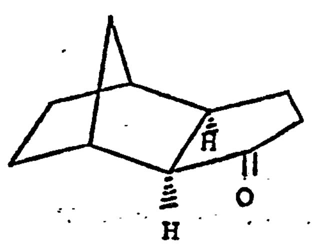

in der die Wasserstoffatome an den C-Atomen 2 und 6 zueinander cis konfiguriert sind und der Cyclopentanonring entweder endo-ständig (Formel IX a und b), oder exo-ständig (Formel X a und b) zum bicyclischen Ringsystem orientiert ist.

Verbindungen der Formel IX a und X a sind aus R. R. Sauer, J. Org. Chem. 39, 1850 (1974) bekannt, und die Verbindungen der Formel IX b und X b sind in J. Org. Chem. 32, 3120 (1967) beschrieben.

Die Ketone IX und X werden nach bekannten Methoden in die Oxime oder Oximderivate der Formel XI überführt

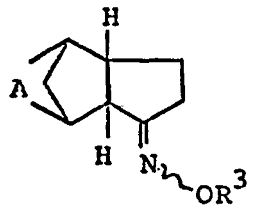

(XI)

in der die H-Atome an den C-Atomen 2 und 6 zueinander cis-konfiguriert sind, der Cyclopentanring endo- oder exoständig zum bicyclischen Ringsystem orientiert ist, A eine CH=CH oder $CH_2$-$CH_2$-Gruppe bedeutet und $R^3$ Wasserstoff, Alkyl, Aryl, Aralkyl, -$SO_3H$, Arylsulfonyl oder eine andere Gruppe, die für die Beckmann-Umlagerung geeignet ist, bedeutet. Bevorzugt ist $R^3$ Wasserstoff, ($C_1$ bis $C_6$)-Alkyl, ($C_6$ bis $C_9$)-Aryl, ($C_7$ bis $C_{10}$)-Aralkyl, $SO_3H$, Benzolsulfonyl und p-Toluolsulfonyl. Die Verbindungen der Formel XI werden in einer Beckmann-Umlagerung, s. Organic Reactions 11 [1960] 1-156, durch Umsatz mit einer Mineralsäure wie beispielsweise Schwefelsäure oder Polyphosphorsäure oder im Falle $R^3$ = H mit Benzolsulfonylchlorid oder p-Toluolsulfonylchlorid und einer Base wie Triäthylamin oder mit einer organischen Säure wie z. B. Ameisensäure in eine Verbindung der Formel XII überführt,

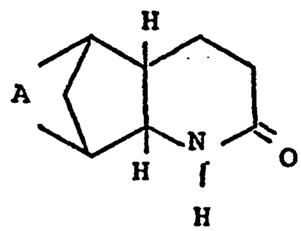

(XII)

in der die H-Atome an den C-Atomen 2 und 7 zueinander cis-konfiguriert sind, der Laktamring endo- oder exoständig zum bicyclischen Ringsystem orientiert ist und A die obengenannte Bedeutung besitzt. Die nach der Beckmann-Umlagerung auftretenden Regioisomeren können leicht durch Umkristallisation oder durch Säulenchromatographie über Kieselgel entfernt werden. Die Verbindungen der Formel XII werden zu einer Verbindung der Formel XIII halogeniert

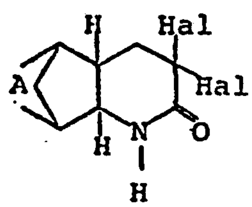

(XIII)

in welcher A die obengenannte Bedeutung besitzt und Hal ein Halogenatom, vorzugsweise Chlor oder Brom bedeutet. Als Halogenierungsmittel kommen beispielsweise anorganische Säurehalogenide wie $PCl_5$, $SO_2Cl_2$, $POCl_3$, $SOCl_2$, $PBr_3$ oder Halogene wie Brom in Betracht. Von Vorteil ist die Verwendung von $PCl_5$ oder $POCl_3$ in Kombination mit $SO_2Cl_2$. Als Zwischenstufe bildet sich zunächst ein Imidhalogenid, das mit den genannten Halogenierungsmitteln und anschließender Hydrolyse unter basischen Bedingungen, vorzugsweise mit wäßrigem Alkalicarbonat weiter zu einer Verbindung der Formel XIII reagiert.

Die Verbindungen der Formel XIII werden nachfolgend in einem polaren protischen Lösungsmittel wie beispielsweise einem Alkohol, vorzugsweise Ethanol, oder einer Carbonsäure wie beispielsweise Essigsäure unter Zusatz eines Säureacceptors wie beispielsweise Natriumacetat oder Triethylamin zu einer Verbindung der Formel XIV

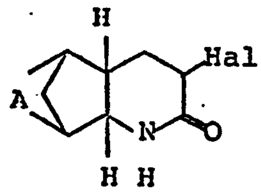

(XIV)

in welcher A und Hal die oben genannten Bedeutungen besitzen, katalytisch reduziert. Als Katalysator kommen beispielsweise Raney-Nickel oder Palladium bzw. Platin auf Tierkohle in Betracht.

Im Falle von A = CH=CH ist es notwendig, die C-C-Doppelbindungen über einen Cyclopentadienyleisendicarbonyl-Komplex nach Formel XV zu schützen. Der Eisenkomplex wird nach der Hydrierung wieder mit Na in Aceton entfernt, wie es in J. Amer. Chem. Soc. 97, 3254 (1975) von K.M. Nicholas beschrieben ist.

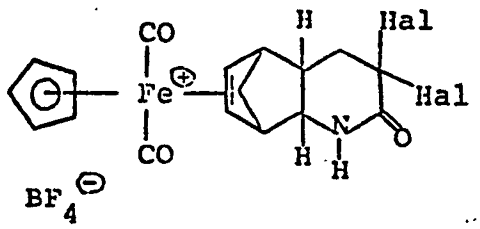

(XV)

Verbindungen der Formel XIV können auch direkt durch Halogenierung der Verbindungen der Formel XII beim Einsatz geringerer Mengen der obengenannten Halogenierungsmittel hergestellt werderen.

Verbindungen der Formel XIV werden gemäß der bekannten Favorskii-Reaktion in Gegenwart einer Base zu einer Verbindung der Formel III mit W = Wasserstoff umgesetzt und diese gegebenenfalls verestert. Die obengenannte Favorskii-Reaktion wird in einem alkoholischen Lösungsmittel wie Methanol, Ethanol oder tert.-Butanol oder in Wasser oder in Gemischen derselben bei Temperaturen im Bereich von 20°C bis 140°C, vorzugsweise zwischen 60°C und 100°C durchgeführt. Als Basen werden zweckmäßig Alkali- oder Erdalkalihydroxide wie Natrium-, Kalium- oder Bariumhydroxid oder Alkalialkoholate wie beispielsweise Natriummethylat oder Kalium-tert.-butanolat eingesetzt.

Die nach der Favorskii-Reaktion erhaltenen Verbindungen der Formel III mit W = Wasserstoff sind Racemate und können als ein Diastereomerengemisch vorliegen. So erhält man ausgehend von den Ketonen der Formel IX die Aminosäuren der Formel IIIa und IIIb zusammen mit den Spiegelbildisomeren und ausgehend vom Keton der Formel X die Aminosäuren der Formel IIIc und IIId mit den dazugehörigen Spiegelbildisomeren mit W = Wasserstoff, wobei A die obige Bedeutung besitzt.

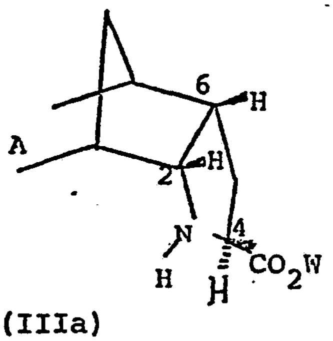

(IIIa)

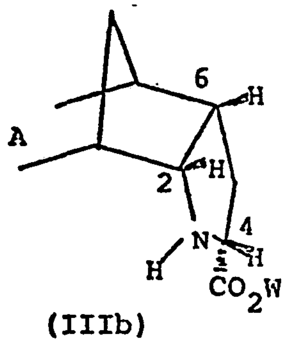

(IIIb)

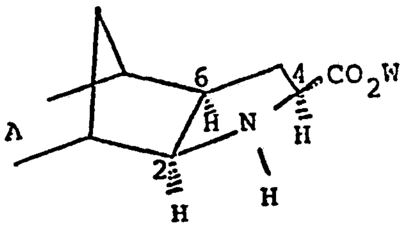

(IIIc)

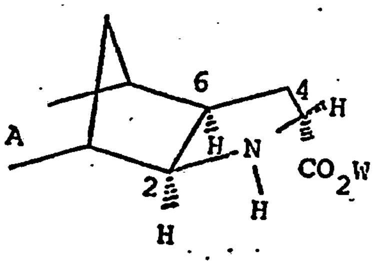

(IIId)

In allen vier Verbindungen der Formeln IIIa-IIId sind die Wasserstoffatome an $C_2$ und $C_6$ zuinander cis-konfiguriert; in Verbindungen der Formel IIIa und IIIb ist der Pyrrolidinring endo-ständig, in den Formeln IIIc und IIId exo-ständig zum bicyclischen Ringsystem orientiert, die -$CO_2$W-Gruppen an C-Atom 4 in den Verbindungen der Formeln IIIa und IIId sind cis-ständig bezüglich des Wasserstoffatoms an C-2 orientiert und in den Verbindungen der Formel IIIb und IIIc entsprechend trans-ständig. In die folgenden Reaktionen können die entsprechenden Racemate oder Diastereomerengemischee eingesetzt werden. Die Racemate können aber vorher auch nach bekannten Methoden der Peptidchemie in die optischen Antipoden getrennt werden, und die Diastereomerengemische können durch Fraktionskristallisation oder nach geeigneter Derivatisierung durch Säulenchromatographie über Kieselgel in die Diastereomeren getrennt werden.

Die Aminosäuren können gegebenenfalls verestert werden. Die bevorzugten tert.-Butylester und Benzylester der Aminosäuren der Formel III (W = tert.-Butyl oder Benzyl) werden nach den in der Peptidchemie üblichen Methoden erhalten, wie z. B. im Falle des tert.-Butylesters durch Reaktion der Säuren mit Isobutylen in einem inerten organischen Lösungsmittel (z. B. Dioxan) in Gegenwart von Säuren (wie z. B. Schwefelsäure). Im Falle von A = CH=CH hat sich als besonders vorteilhaft das folgende Verfahren erwiesen: Die entsprechende Aminosäure wird mit einer basisch abspaltbaren Gruppe, wie z. B. der Methylsulfonylethoxycarbonylgruppe (= MSC), Tesser, Balvert-Geers, Int. J. Pept. Protein Res. 7, 295 (1975) am Stickstoff acyliert.

Die Carbonsäure wird im neutralen bis schwach basischen pH-Bereich mit tert.-Butanol in einem organischen Lösungsmittel, wie z. B. Pyridin, in Gegenwart von Propylphosphonsäureanhydrid zum entsprechenden tert.-Butylester umgesetzt. Durch Abspaltung der MSC-Schutzgruppe im stark alkalischen pH-Bereich mit Alkali im wässrigen Lösungsmittel wird der tert.-Butylester der Formel III erhalten (W = tert.-Butyl).

Die Benzylester der Formel II (W = Benzyl) werden nach der üblichen Methode mit Benzylalkohol und Thionylchlorid erhalten.

Die als Ausgangsstoffe zur Herstellung der Verbindungen der Formel I verwendeten Verbindungen der Formel II mit n = 1, Y, Z = Wasserstoff, $R^1$ = Methyl und $R^2$ = Methyl oder Ethyl und X = Phenyl sind bekannt (europäische Patentanmeldung Nr. 37 231). Die Verbindungen der Formel II lassen sich nach verschiedenen Verfahrensweisen herstellen. Eine Synthesevariante geht von einem Keton der oben genannten Formel VII aus, das nach bekannten Verfahrensweisen in einer Mannich-Reaktion mit einer Verbindung der oben genannten Formel VI zusammen mit Aminosäureestern der Formel XVI

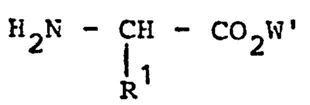

(XVI)

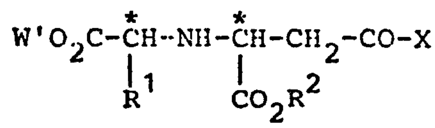

(XVII)

worin $R^1$ die oben genannte Bedeutung besitzt und W' einen hydrogenolytisch oder sauer abspaltbaren Rest, insbesondere einen Benzyl- oder einen tert.-Butyl-Rest, bedeutet, zu einer Verbindung der Formel XVII, worin $R^1$, $R^2$, X und W' die oben genannten Bedeutungen besitzen, mit der Einschränkung, daß, wenn W' einen hydrogenolytisch abspaltbaren Rest, insbesondere Benzyl, bedeutet, $R^2$ nicht die Bedeutung von W' besitzen darf, umsetzt. Spaltet man den Rest W' hydrogenolytisch mit Hilfe von beispielsweise Palladium ab, werden bei einer Wasserstoffaufnahme von 3 Moläquivalenten Vorbindungen der Formel II mit Y, Z = Wasserstoff erhalten. Stoppt man die Wasserstoffaufnahme bei 1 Moläquivalent, erhält man Verbindungen der Formel II mit n = 1 und Y und Z zusammen = Sauerstoff, die man ebenfalls erhält, wenn der Rest W' der Formel XVII mit Säuren, wie beispielsweise Trifluoressigsäure oder Salzsäure, in einem inerten organischen Lösungsmittel, wie beispielsweise Dioxan, abgespalten wird.

Verbindungen der Formel XVII sind auch durch Michael-Additionen einer Verbindung der oben genannten Formel V mit einer Verbindung der oben genannten Formel XVI nach bekannten Verfahrensweisen zugänglich. Bevorzugt eignet sich dieses Verfahren zur Herstellung von solchen Verbindungen der Formel XVII, in denen $R^1$ = Methyl, $R^2$ = Ethyl und X = Aryl bedeuten.

Die Verbindungen der Formel XVII fallen als Diastereomerengemische an. Bevorzugte Diastereomere der Formel XVII sind solche, in denen die mit einem Stern markierten chiralen C-Atome jeweils S-Konfiguration aufweisen. Diese können beispielsweise durch Kristallisation oder durch Chromatographie, z. B. an Kieselgel, abgetrennt werden. Bei der nachfolgenden Abspaltung des Restes W' bleiben die Konfigurationen der chiralen C-Atome erhalten.

Die als Ausgangsstoffe zur Herstellung der Verbindungen der Formel I verwendeten Verbindungen der oben genannten Formel IV werden aus den Verbindungen der oben genannten Formel III durch Umsetzen mit einer N-geschützten 2-Aminocarbonsäure der Formel XVIII

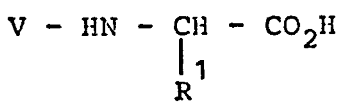

(XVIII)

wohin V eine Schutzgruppe und $R^1$ die oben genannte Bedeutung besitzt, nach bekannten Verfahrensweisen erhalten. Als Schutzgruppe V, die nach beendeter Reaktion wieder abgesplaten wird, kommt beispielsweise tert.-Butoxycarbonyl oder Benzyloxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden sauren Abspaltung des Restes W werden als Säuren bevorzugt Trifluoressigsäure oder Chlorwasserstoff eingesetzt. Die Abspaltung der Benzylgruppe (W = Benzyl), erfolgt bevorzugt durch Hydrogenolyse an Palladium auf Kohle in Alkohol.

In den oben beschriebenen Reaktionen zur Herstellung der Verbindungen der Formeln III, IV und I bleiben jeweils die Konfigurationen der Zwischenprodukte an den Brückenkopf C-Atomen 2 und 6 erhalten.

Die gemäß oben beschriebener Verfahrensweise erhaltenen Verbindungen der Formel III fallen als Gemisch an und können beispielsweise durch Umkristallisation oder durch Chromatographie voneinander getrennt werden.

Die Verbindungen der Formel III fallen als racemische Gemische an und können als solche in die weiteren oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantiomere eingesetzt werden. Die reinen Enantiomeren können auch erhalten werden.

Fallen die Verbindungen der Formel I als Racemate an, können auch diese nach den üblichen Methoden wie beispielsweise über Salzbindung mit optisch aktiven Basen oder Säuren in ihre Enantiemeren gespalten oder durch Chromatographie getrennt werden.

Die erfindungsgemäßen Verbindungen der Formel I, liegen, falls R = Wasserstoff ist, als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit

anderen blutdrucksenkenden, gefäßerweiternden oder diuretisch wirksamen Verbindungen ist möglich. Typische Vertreter dieser Wirkklassen sind z. B. in Erhardt-Ruschig, Arzneimittel, 2. Auflage, Weinheim 1972, beschrieben. Die Anwendung kann intravenös, subcutan oder peroral erfolgen.

Die Dosierung bei oraler Gabe liegt bei 1 - 100 mg, vorzugsweise bei 1 - 40 mg je Einzeldosis bei einem normalgewichtigen erwachsenen Patienten; dies entspricht ca. 0,013-1,3 Tg/kg/Tag, vorzugsweise 0,013-0,53 mg/kg/Tag. Die Dosis kann in schweren Fällen auch erhöht werden, da toxische Eigenschaften bisher nicht beobachtet wurde. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebrächt, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propandiol oder Glycerin, daneben auch Zuckerlösungen wie Glukose- oder Mannitlösungen, oder auch eine Mischung aus den verschiedenen genannten Lösungsmitteln.

Die nachfolgenden Beispiele dienen zur Erläuterung der Erfindung, ohne diese auf die stellvertretend genannten Verbindungen zu beschränken.

Die in den folgenden Beispielen angegebenen $^1$H-NMR-Daten wurden, wenn nicht anders angegeben, durch Messung in $CDCl_3$ ermittelt und sind in δ(ppm) angegeben.

**Beispiel 1**

N-(1-S-Carbethoxy-3-phenyl-propyl)-S-alanyl-1R,2R,4S,6S,7S -tricyclo[5.2.1.0$^{2.6}$]-3-aza-decan-4-carbonsäure-hydrochlorid

a) (±) Endo-tricyclo[6.2.1.0$^{2.7}$]-3-aza-4-oxo-undecan

7.8 g (±) Endo-tricyclo[5.2.1.0$^{2.6}$]-3-oxo-decan (Journ. Org. Chem. 32, 3120, 1967) werden in 52 ml 95 %iger Ameisensäure gelöst. Dazu werden 9.1 g Hydroxylamin-O-sulfonsäure, die in 26 ml 95 %iger Ameisensäure gelöst ist, innerhalb von 10 min zugetropft. Danach wird 2 Stunden am Rückfluß gekocht. Nach dem Abkühlen wird mit Eis versetzt und unter Eiskühlung mit konz. Natronlauge neutralisiert. Es wird mit Methylenchlorid extrahiert, der Extrakt mit Wasser gewaschen, getrocknet und einrotiert. Rohausbeute 7.4 g Gemisch. Das Gemisch, das aus endo-tricyclo[6.2.1.0$^{2.7}$]-3-aza-4-oxo-undecan und endo-tricyclo[6.2.1.0$^{2.7}$]-4-aza-3-oxo, undecan besteht wird über Kieselgel mit Methylenchlorid/Methanol 95:5 getrennt.

Ausbeute: 4.5 g Fp. 170°-172°C

b) (±) Endo-tricyclo[6.2.1.0$^{2.7}$]-3-aza-4-oxo-5,5-dichlor-undecan

4.5 g Laktam aus Beispiel 1a werden in 70 ml wasserfreiem Chloroform gelöst und unter Rühren bei Raumtemperatur mit 5.6 g Phosphorpentachlorid versetzt. Zu diesem Gemisch werden 7.8 g Sulfurylchlorid in 6 ml Chloroform innerhalb von 30 min zugetropft und dann 3 Stunden refluxiert. Anschließend wird unter Kühlung mit gesättigter Kaliumcarbonatlösung neutral gestellt. Nach dem Abtrennen der Chloroformphase wird die wässrige Phase mit Methylenchlorid extrahiert, die organische Phasen werden vereinigt, mit Wasser gewaschen, getrocknet und im Vakuum eingeengt. Das Rohprodukt wird über Kieselgel mit Methylenchlorid/Methanol 95:5 filtriert.

Ausbeute: 3.7 g Fp: 199-200°C

c) (±)-Endo-tricyclo[6.2.1.0$^{2.7}$]-3-aza-4-oxo-5-chlor-undecan

2.9 g Dichlorlaktam aus Beispiel 1b und 1.7 ml Triethylamin werden in 170 ml Ethanol gelöst. Dazu werden etwa 0.7 g Raney-Nickel gegeben und hydriert. Nach der Aufnahme von 1 Äquivalent Wasserstoff wird abgebrochen, vom Katalysator abgesaugt und die Ethanollösung im Vakuum eingeengt. Der Rückstand wird in Essigester aufgenommen, mit Wasser gewaschen, getrocknet und eingeengt. Der Rückstand wird über Kieselgel mit Methylenchlorid/Methanol 95:5 getrennt.

Ausbeute: 1.7 g, Fp: 179-181°C

d) 1:1 Gemisch bestehend aus 1R,2R,4S,6S,7S -Tricyclo[5.2.1.0$^{2.6}$]-3-aza-decan-4-carbonsäure und 1S,2S,4R,6R,7R-Tricyc1o[5.2.1.0$^{2.6}$]-3-aza-decan-4-carbonsäure

1.3 g Monochlorlaktam aus Beispiel 1c werden zu einer siedenden Lösung von 2.2 g Bariumhydroxid-Octahydrat in 39 ml Wasser gegebeh. Es wird 4 Stunden refluxiert, danach mit konz. Schwefelssure auf pH 6.5 eingestellt und 1 Stunde refluxiert. Nach dem Abkühlen wird vom Niederschlag abgesaugt. Die Mutterlauge

wird zur Trockene engeengt und der Rückstand aus Essigester kristallisiert.

Ausbeute: 1.1 g, $R_f$: 0.61 ($SiO_2$; $CH_2Cl_2/CH_3OH/CH_3CO_2H/H_2O$ 20:15:2:4)

Bei der Reaktion entsteht laut $^1$H-NMR (270 MHz) ein kleiner Anteil an 1S,2S,4S,6R,7R-Tricyclo[5.2.1.$0^{2.6}$]-3-aza-decan-4-carbonsäure und 1R,2R,4R,6S,7S-Tricyclo[5.2.1.$0^{2.6}$] -3-aza-decan-4-carbonsäure, der abgetrennt werden kann.

e) 1:1 Gemisch bestehend aus 1R,2R,4S,6S,7S-Tricyclo[5.2.1.$0^{2.6}$]-3-aza-decan-4-carbonsäure-benzylesterhydrochlorid und 1S,2S,4R,6R,7R-Tricyclo[5.2.1.$0^{2.6}$]-3-aza-decan-4-carbonsäure-benzylesterhydrochlorid

10 ml Benzylalkohol werden auf -5°C abgekühlt und 1.7 g Thionylchlorid zugetropft. Zu dieser Lösung werden 1.1 g racemische Aminosäure aus Beispiel 1d zugegeben. Man läßt auf 0°C kommen und rührt 17 Stunden bei 5°C. Der Benzylalkohol wird im Vakuum abdestilliert und der Rückstand mit Diisopropyläther verrieben.

Ausbeute 1.1 g

$^1$H-NMR (DMSO-$d_6$) : 1.0-3.0 (m, 5H), 3.2-4.8 (m, 8H) 5.2 (s, 2H), 7.4 (br. s 5H), 9.2-10.5 (breit, 2H)

f) N-(1-S-Carbethoxy-3-phenyl-propyl)-S-alanyl-1R,2R,4S,6S,7S-tricyclo[5.2.1.$0^{2.6}$]-3-aza-decan-4-carbonsäure-benzylester

In 10 ml wasserfreiem Dimethylformamid werden nacheinander unter Eiskühlung 0.96 g N-(1-S-Carbethoxy-3-phenyl-propyl)-S-alanin, 0.46 g Hydroxybenztriazol, 1 g Benzylester aus Beispiel 1e, 0.7 g Dicyclohexylcarbodiimid und 0.4 g N-Äthylmorpholin gegeben und läßt 17 Stunden bei Raumtemperatur rühren. Es wird dann mit 12 ml Essigester verdünnt und der ausgefallene Harnstoff abgesaugt. Das Lösungsmittel wird im Vakuum abdestilliert. Der Rückstand wird in Äther aufgenommen, mit gesättigter Natriumcarbonatlösung und Wasser gewaschen, getrocknet und eingeengt. Der Rückstand 1.7 g wird über Kieselgel mit Cyclohexan/Essigester 8:2 in die einheitlichen Diastereomeren getrennt.

Die schnell laufende Fraktion enthält N-(1-S-Carbethoxy-3-phenyl-propyl)-S-alanyl-1S,2S,4R,6R,7R-tricyclo-[5.2.1.$0^{2.6}$]-3-aza-decan-4-carbonsäure-benzylester 100 mg; m/e: 532 $R_f$ = 0.52-($SiO_2$; Cyclohexen/ Essigester 1:1)

Die langsam laufende Fraktion enthält 400 mg N-(1-S-Carbethoxy-3-phenyl-propyl)-S-alanyl-1R, 2R, 4S, 6S, 7S-tricyclo[5.2.1.$0^{2.6}$]-3-aza-decan-4-carbonsäure-benzylester

m/e : 532; $R_f$ = 0.43 ($SiO_2$; Cyclohexan/Essigester 1:1)

g) N-(1-S-Carbethoxy-3-phenyl-propyl)-5-alanyl-1R,2R,4S,6S,7S-tricyclo[5.2.1.$0^{2.6}$]-3-aza-decan-4-carbonsäure-hydrochlorid

350 mg N-(1-S-Carbethoxy-3-phenyl-propyl)-S-alanyl-1R,2R,4S,6S,7S-tricyclo[5.2.1.$0^{2.6}$]-3-aza-decan-4-carbonsäurebenzylester aus Beispiel 1f werden in 10 ml Ethanol gelöst, mit 30 mg 10 % Palladium auf Kohle versetzt und bei Raumtemperatur hydriert. Nach dem Absaugen vom Katalysator wird die Lösung im Vakuum eingeengt, der Rückstand in Essigester gelöst, die Lösung mit ethanolischem Chlorwasserstoff sauer gestellt und einrotiert. Der Rückstand wird mit Diisopropyläther verrieben.

Ausbeute: 270 mg Fp: 162-165°C (Zers.)

$R_f$ =0.42 ($SiO_2$; Methylenchlorid / Methanol 8:2)

**Beispiel 2**

N-(1-S-Carbethoxy-3-phenyl-propyl)-S-alanyl-1S,2S,4S,6R,7R-tricyclo[5.2.1.$0^{2.6}$]-3-aza-decan-4-carbonsäure-hydrochlorid

In analoger Weise wird nach dem in Beispiel 1e bis 1g beschriebenen Verfahren mit 1S,2S,4S,6R,7R-Tricyclo-[5.2.1.$0^{2.6}$]-3-aza-decan-4-carbonsäure als Ausgangsprodukt N-(1-S-Carbethoxy-3-phenyl-propyl)-S-alanyl-1S,2S,4S,6R,7R-tricyclo[5.2.1.$0^{2.6}$]-3-aza-decan-4-carbonsäure-hydrochlorid erhalten.

**Beispiel 3**

N-(1-S-Carbethoxy-3-phenyl-propyl)-S-alanyl-1S,2R,4S,6S,7R-tricyclo[5.2.1.$0^{2.6}$]-3-aza-decan-4-carbonsäure

a) (±) Exo-tricyclo[6.2.1.$0^{2.7}$]-3-aza-4-oxo-undecan

Die Verbindung wird ausgehend von (±) Exo-tricyclo-[5.2.1.$0^{2.6}$]-3-oxo-decan (Journ. Org. Chem 32, 3120 (1967)) nach dem im Beispiel 1a) beschriebenen Verfahren hergestellt. Das Laktam-Gemisch wird über Kieselgel mit Methylenchlorid/Methanol 9:1 getrennt. $R_f$ 0-49 ($SiO_2$; $CH_2Cl_2/CH_3OH$ 9:1) Fp: 178-180°C

b) (±) Exo-tricyclo[6.2.1.$0^{2.7}$]-3-aza-4-oxo-5.5-dichlor-undecan

Die Verbindung wird ausgehend vom Laktam des Beispiel 3a nach dem in Beispiel 1b beschriebenen Verfahren

Fp: 240°C, $R_f$ 0.49 ($SiO_2$; $CH_2Cl_2/CH_3OH$ 95:5)

c) (±) Exo-tricyclo[6.2.1.$0^{2.7}$]-3-aza-4-oxo-5-chlor-undecan.

Die Verbindung wird ausgehend vom Dichlorlaktam des Beispiel 3b nach dem in Beispiel 1c beschriebenen Verfahren hergestellt.

$R_f$ = 0.26 ($SiO_2$; $CH_2Cl_2$ / $CH_3OH$ 95:5)

d) 1:1 Gemisch bestehend aus 1S,2R,4S,6S,7R-Tricyclo[5.2.1.$0^{2.6}$]-3-aza-decan-4-carbonsäure und

Spiegelbildisomeres und 1R,2S,4S,6R,7S-Tricyclo[5.2.1.$0^{2.6}$]-3-aza-decan-4-carbonsäure und Spiegelbildisomeres.

Die Verbindungen werdenausgehend vom Monochlorlaktam des Beispiel 3c nach dem in Beispiel 1d beschriebenen Verfahren hergestellt.

$R_f$ = 0.54 ($SiO_2$; $CH_2Cl_2$ / $CH_3OH$/$CH_3CO_2H$/ $H_2O$ 20:15:2:4)

NMR ($D_2O$) : 0.9-1.6 (m, 8H); 2.1-2.5 (m, 3H); 3.4-4.0 (m, 2H)

e) 1:1-Gemisch bestehend aus 1S,2R,4S,6S,7R-Tricyclo[5.2.1.$0^{2.6}$]-3-aza-decan-4-carbonsäure-benzylester und Spiegelbildisomeres und 1R,2S,4S,6R,7S-Tricyclo-[5.2.1.$0^{2.6}$]-3-aza-decan-4-carbonsäure-benzylester und Spiegelbildisomeres

Die Verbindungen werden ausgehend von dem Aminosäuregemisch des Beispiel 3d nach dem in Beispiel 1e beschriebenen Verfahren hergestellt.

$R_f$ = 0.31 Diastereomer I ($SiO_2$; $CH_2Cl_2$ / $CH_3OH$ 95:5)

$R_f$ = 0.26 Diastereomer II

Das Gemisch kann nach N-Acylierung über Kieselgel chromatographisch in die beiden racemischen Diastereomere präparativ getrennt werden.

f) Gemisch aus N-(1-S-Carbethoxy-3-phenyl-propyl)-S-alanyl-1S,2R,4S,6S,7R-tricylo[5.2.1.$0^{2.6}$]-3-aza-decan-4-carbonsäurebenzylester, N-(1-S-Carbethoxy-3-phenyl-propyl)-S-alanyl-1R,2S,4R,6R,7S-tricyclo-[5.2.1.$0^{2.6}$]-3-aza-decan-4-carbonsäurebenzylester, N-(1-S-Carbethoxy-3-phenyl-propyl)-S-alanyl-1R,2S,4S,6R,7S-tricyclo-[5.2.1.$0^{2.6}$]-3-aza-decan-4-carbonsärurebenzylester und N-(1-S-Carbethoxy-3-phenyl-propyl)-S-alanyl-1S,2R,4R,6S,7R-tricyclo-[5.2.1.$0^{2.6}$]-3-aza-decan-4-carbonsäure-benzylester

Die Verbindungen werden ausgehend vom Benzylestergemisch des Beispiel 3e nach dem in Beispiel 1f beschriebenen Verfahren hergestellt. Das Diastereomerengemisch wird über Kieselgel mit Methylenchlorid/Essigester 99:1 bis 8:2 getrennt.

Diastereomer A

$R_f$ = 0.134 ($SiO_2$; Methylenchiorid / Essigester 95:5) m/e = 532

Diastereomer B

$R_f$ = 0.126 ($SiO_2$; Methylenchlorid / Essigester 95:5) m/e = 532

Diastereomer C

$R_f$ = 0.105 ($SiO_2$; Methylenchlorid /Essigester 95:5) m/e = 532

Diastereomer D

$R_f$ = 0.074 ($SiO_2$; Methylenchlorid /Essigester 95:5) m/e = 532

g) N-(1-S-Carbethoxy-3-phenyl-propyl)-S-alanyl-1S, 2R,4S, 6S, 7R-tricyclo[5.2.1.$0^{2.6}$]-3-aza-decan-4-carbonsäure-hydrochlorid

N-(1-S-Carbethoxy-3-phenyl-propyl)-S-alanyl-1R,2S,4R,6R,7S-tricyclo[5.2.1.$0^{2.6}$]-3-aza-decan-4-carbonsäure--hydrochlorid

N-(1-S-Carbethoxy-3-phenyl-propyl)-S-alanyl-1R,2S,4S,6R,7S-tricyclo[5.2.1.$0^{2.6}$]-3-aza-decan-4-carbonsäure--hydrochlorid,

N-(1-S-Carbethoxy-3-phenyl-propyl)-S-alanyl-1S,2R,4R,6S,7R-tricyclo[5.2.1.$0^{2.6}$]-3-aza-decan-4-carbonsäure--hydrochlorid entstehen, wenn die Diastereomeren A, B, C, D des Beispiel 3g nach dem in Beispiel 1g beschriebenen Verfahren jeweils umgesetzt werden.

Diastereomer A'

$R_f$ = 0.191 ($SiO_2$; Methylenchlorid / Methanol 9:1) m/e : 514 als Trimethylsilylderivat

Diastereomer B'

$R_f$ = 0.231 ($SiO_2$; Methylenchlorid / Methanol 9:1) m/e: 514 als Trimethylsilylderivat

Diastereomer C'

$R_f$ = 0.301 ($SiO_2$; Methylenchlorid / Methanol 9:1) m/e: 514 als Trimethylsilylderivat

Diastereomer D'

$R_f$ = 0.358 ($SiO_2$; Methylenchlorid / Methanol 9:1) m/e: 514 als Trimethylsilylderivat

**Beispiel 4**

N-(1-S-Carboxy-3-phenyl-propyl)-S-alanyl-1R,2R,4S,6S,7S-tricyclo[5.2.1.$0^{2.6}$]-3-aza-decan-4-carbonsäure 1 g des Esters aus Beispiel 1g werden in 20 ml Dimethoxyethan gelöst. Man gibt einen Tropfen einer verdünnten Indikaterlösung, z. B. Bromthymolblau zu und fügt unter starkem Rühren im Verlauf von 5 Minuten eine äquivalente Menge 4 n wässriger Kaliumhydroxidlösung zu, so daß der Indikator bei Beendigung der Reaktion einen pH-Wert von 9-10 zeigt. Danach stellt man mit Salzsäure auf pH 4, engt im Vakuum zur Trockne ein, nimmt in Essigester auf und filtriert. Nach dem Einengen der Essigesterlösung erhält man 0.75 g eines festen Rückstandes.

m/e : 414

**Beispiel 5**

N-(1-S-Carboxy-3-phenyl-propyl)-S-alanyl-1S,2R,4S,6S,7R-tricyclo[5.2.1.$0^{2.6}$]-3-aza-decan-4-carbonsäure
N-(1-S-Carboxy-3-phenyl-propyl)-S-alanyl-1R,2S,4R,6R,7S-tricyc1o[5.2.1.$0^{2.6}$]-3-aza-decan-4-carbonsäure
N-(1-S-Carboxy-3-phenyl-propyl)-S-alanyl-1R,2S,4S,6R,7S-tricyclo[5.2.1.$0^{2.6}$]-3-aza-decan-4-carbonsäure
N-(1-S-Carboxy-3-phenyl-propyl)-S-alanyl-1S, 2R, 4R, 6S, 7R-tricyclo[5.2.1.$0^{2.6}$]-3-aza-decan-4-carbonsäure
entstehen nach der Verseifung, wie sie in Beispiel 4 beschrieben ist, aus den Diastereomeren A', B', C' und D' des Beispiel 3g.

**Beispiel 6**

N-(1-S-Carbethoxy-3-oxo-3-phenyl-propyl)-S-alanin-benzyl-ester
65,7 g 4-Phenyl-4-oxo-buten-2-carbonsäure-ethylester (Benzoylacrylsäureethylester) werden in 225 ml Ethanol gelöst und dazu 1 ml Triethylamin gegeben. Zu dieser Lösung wird bei Raumtemperatur eine Lösung von 70 g S-Alaninbenzylester in 90 ml Ethanol rasch zugetropft. Es wird 2 Stunden bei Raumtemperatur gerührt und dann dio Lösung abgekühlt. Es kristallisiert das S,S-Isomere aus.
Ausbeute: 94,3 g Fp.: 73-74° C
$[\alpha]_D^{20} = + 17.8°$ (c = 1, $CH_3OH$)

**Beispiel 7**

N-(1-S-Carbethoxy-3-oxo-3-phenyl-propyl)-S-alanin 0,5 g der Verbindung aus Beispiel 6 werden in 40 ml Ethanol gelöst und 0,1 g Pd/C 10 %ig zugegeben und bei Raumtemperatur und Normaldruck hydriert.
Ausbeute: 300 mg Fp.: 210-220° C
$^1$H-NMR (DMSO-$d_6$):
1,0-1,4 (t, 6H); 3,2-5,0 (m, 8H); 7,2-8,2 (m, 5H)

**Beispiel 8**

N-(1-S-Carbethoxy-3-oxo-3-phenyl-propyl)-S-alanyl-1R,2R,4S,6S,7S-tricyclo[5.2.1.$0^{2.6}$]-3-aza-decan-4-carbonsäure-benzylester
Die Verbindung wird aus dem Benzylestergemisch des Beispiel 1e und N-(1-S-Carbethoxy-3-oxo-3-phenylpropyl-S-alanin aus Beispiel 7 analog dem Verfahren, das in Beispiel 1f beschrieben ist, hergestellt. Die Diastereomeren werden über Kieselgel getrennt.

**Beispiel 9**

N-(1-S-Carbethoxy-3-oxo-3-phenyl-propyl)-S-alanyl-1R,2R,4S,6S,7S -tricyclo[5.2.1.$0^{2.6}$]-3-aza-decan-4-carbonsäure
1 g des Benzylesters aus Beispiel 8 werden in 30 ml Ethanol gelöst, mit 100 mg Pd/C (10 %ig) bei Raumtemperatur und Normaldruck hydriert. Nach der Aufnahme eines Moläquivalentes Wasserstoff wird die Hydrierung abgebrochen. Es wird vom Katalysator abgesaugt, die Lösung eingeengt.
Ausbeute: 750 mg eines Öls
Analog werden nach den in den Beispielen 8 und 9 beschriebenen Verfahren ausgehend von den Aminosäurebenzylestern des Beispiel 3e die Verbindungen hergestellt:
N-(1-S-Carbethoxy-3-oxo-3-phenyl-propyl)-S-alanyl-1S,2R,4S,6S,7R-tricyclo[5.2.1.$0^{2.6}$]-3-aza-decan-4-carbonsäure
N-(1-S-Carbethoxy-3-oxo-3-phenyl-propyl)-S-alanyl-1R,2S,4R,6R,7S -tricyclo[5.2.1.$0^{2.6}$]-3-aza-decan-4-carbonsäure
N-(1-S-Carbethoxy-3-oxo-3-phenyl-propyl)-S-alanyl-1R,2S,4S,6R,7S-tricyc1o[5.2.1.$0^{2.6}$]-3-aza-decan-4-carbonsäure
N-(1-S-Carbethoxy-3-oxo-3-phenyl-propyl)-S-alanyl-1S,2R,4R,6S,7R-tricyclo[5.2.1.$0^{2.6}$]-3-aza-decan-4-carbon-säure

**Beispiel 10**

$N_{\alpha}$-(1-S-Carbethoxy-3-phenyl-propyl)-S-lysyl-1R,2R,4S,6S,7S-tricyclo[5.2.1.0$^{2.6}$]-3-aza-decan-4-carbonsäure-dihydrochlorid

a) $N_{\alpha}$-(1-S-Carbethoxy-3-oxo-3-phenyl-propyl)-$N_{\varepsilon}$-benzyl-oxycarbonyl-S-lysinbenzylester

10 g 4-phenyl-4-oxo-buten-2-carbonsäure-ethylester werden in 100 ml Ethanol gelöst. Dazu werden 19,1 g $N_{\varepsilon}$ -benzyloxycarbonyl-S-lysinbenzylester und 0,2 g Triethylamin gegeben. Die Lösung wird 3 Stunden bei Raumtemperatur gerührt, danach im Vakuum eingeengt. Der ölige Rückstand (31 g) wird in Isopropanol/Diisopropylether gelöst und abgekühlt. Es kristallisieren 13 g $N_{\alpha}$-(1-S-Carbethoxy-3-oxo-3-phenyl-propyl)-$N_{\varepsilon}$-benzyloxycarbonyl-S-lysinbenzylester.

$\alpha_D^{20} = 3{,}5°$ (c = 1, $CH_3OH$)

$^1$H-NMR ($CDCl_3$):

1,0-1,4 (tr, 3H); 1,0-2,0 (m, 9H); 2,0-2,6 (breites s, 1H); 2,9-3,9 (m, 6H); 3,9-4,4 (q, 2H); 4,6-4,9 (breites s, 1H); 5,0-5,2 (doppeltes s, 4H); 7,1-8,1 (m, 15H)

b) $N_{\alpha}$-(1-S-Carbethoxy-3-phenyl-propyl)-$N_{\varepsilon}$-benzyloxy-carbonyl-S-lysin

4,0 g des in Beispiel 10a hergestellten Lysinbenzylesterderivates werden in 50 ml Eisessig gelöst, dazu 0,6 g Pd/C (10%ig) und 0,6 g konz. Schwefelsäure gegeben. Es wird 6 Stunden bei Raumtemperatur und unter Normaldruck hydriert. Danach wird vom Katalysator abgesaugt, die ethanolische Lösung mit 1,4 g festem Natriumhydrogencarbonat gerührt. Die Lösung wird einrotiert und der Rückstand in Wasser gelöst. Die wäßrige Phase wird mit Essigester und Methylenchlorid extrahiert. Die organischen Phasen werden verworfen und die wäßrige Phase im Vakuum zur Trockne eingedampft. Der Rückstand wird mit Methanol ausgerührt. Nach dem Abdampfen des Methanol bleibt ein öliger Rückstund, der bei Behandlung mit Diisopropylether fest wird. Ausbeute an $N_{\alpha}$-(1-S-Carbethoxy-3-phenyl-propyl)-S-lysin: 2,0 g $^1$H-NMR ($D_2O$): 1,0-1,4 (tr, 3H); 1,0-2,5 (m, 9H), 2,5-4,4 (m, 9H); 3,9-4,4 (q, 2H), 4,5-5,0 (m, 1H); 7,1-7,6 (m, 5H)

m/e: 336

3,4 g $N_{\alpha}$-(1-S-Carbethoxy-3-phenyl-propyl)-S-lysin werden in 30 ml Methylenchlorid gelöst und auf 0°C abgekühlt. Dazu werden unter Eiskühlung 2,1 g Triethylamin gegeben und anschließend 1,9 g Chlorameisenbenzylester zugetropft. Es wird 1 Stunde bei 0°C gerührt und dann auf Raumtemperatur gebracht. Die Methylenchloridlösung wird anschließend mit Wasser, Natriumcarbonatlösung und Wasser ausgeschüttelt. Nach dem Trocknen wird eingeengt und der ölige Rückstand über Kiesegel mit Methylenchlorid/Methanol chromatographiert. Es werden 2,0 g $N_{\alpha}$-(1-S-Carbethoxy-3-phenyl-propyl)-$N_{\varepsilon}$-benzyloxycarbonyl-S-lysin erhalten. $^1$H-NMRR ($D_2O$): 1,0-1,4 (tr, 3H); > k 1,0-2,5 (m, 9H); 2,5-4,4 (m, 9H); 3,9-4,4 (q, 2H); 4,5-5,0 (m, 1H); 5,1 (s, 2H1; 7,1-7,5 (m, 10H)

c) $N_{\alpha}$-(1-S-Carbethoxy-3-phenyl-propyl)-$N_{85}$-benzyloxy-carbonyl-S-lysyl-1R,2R,4S,6S,7S-tricyclo[5.2.1.0$^{2.6}$]-3-aza-decan-4-carbonsäure-benzylester

500 mg Benzylesterhydrochlorid, hergestellt in Beispiel 1e, werden mit 900 mg $N_{\alpha}$-(1-S-Carbethoxy-3-phenyl-propyl)-$N_{\varepsilon}$-benzyloxycarbonyl-S-lysin, hergestellt nach Beispiel 10 b, analog Beispiel 1f umgesetzt. Man erhält nach der Aufarbeitung 1,5 g Öl, das ein Gemisch zweier diastereomerer Verbindungen ist.

Das Diastereomerengemisch wird säulenchromatographisch mit Kieselgel und Cyclohexan/Essigester 2:1 als Elutionsmittel in die Einzelkomponenten getrennt. Das zuerst eluierte Isomere stellt obige Verbindung dar. Es werden 0,6 g Öl erhalten.

$^1$H-NMR ($CDCl_3$, nach H/D-Austausch mit $D_2O$): 0,9-3,1 (m, 18H); 3,2-5,1 (m, 14H); 5,1-5,3 (ds, 4H); 7,1-7,6 (m, 15H)

d) $N_{\alpha}$-(1-S-Carbethoxy-3-phenyl-propyl)-S-lysyl-1R,2R,4S,6S,7S-tricyclo[5.2.1.0$^{2.6}$]-3-aza-decan-4-carbonsäure-dihydrochlorid

500 mg $N_{\alpha}$-(1-S-Carbethoxy-3-phenyl-propyl)-$N_{\varepsilon}$-benzyl-oxycarbonyl-S-lysyl-1R,2R,4S,6S,7S-tricyclo[5.2.1.0$^{2.6}$]-3-aza-decan-4-carbonsäurebenzylester aus Beispiel 10c werden in 20 ml Ethanol gelöst und unter Zusatz von 0,1 g 10 %ig Pd/C bei Normaldruck hydrogenolytisch entbenzyliert. Nach Beendigung der Wasserstoffaufnahme wird vom Katalysator abfiltriert, die ethanolische Lösung mit ethanolischer Chlorwasserstofflösung bis pH 1 versetzt, das Ethanol im Vakuum abgedampft. Der Rückstand wird mit Diisopropylether versetzt, wobei das Produkt fest wird. Es werden 200 mg erhalten.

NMR ($D_2O$): 1,0-3,1 (m, 18H); 3,1-5,2 (m, 14H); 7,2 (s, 5H)

**Beispiel 11**

Analog werden nach dem in Beispiel 10c beschriebenen Verfahren durch Umsetzung der Aminosäurebenzylester des Beispiels 3e mit $N_{\alpha}$-(1-S-Carbethoxy-3-phenyl-propyl)-$N_{\varepsilon}$-benzyloxycarbonyl-S-lysin, beschrieben in Beispiel 10b, die folgenden Verbindungen erhalten:

$N_{\alpha}$-(1-S-Carbethoxy-3-phenyl-propyl)1-$N_{\varepsilon}$-benzyloxycarbonyl-S-lysyl-1S,2R,4S,6S,7R-tricyclo[5.2.1.0$^{2.6}$]-3-aza-decan-4-carbonsäure-benzylester (Diastereomer E)

$N_{\alpha}$-(1-S-Carbethoxy-3-phenyl-propyl)-$N_{\varepsilon}$-benzyloxycarbonyl-S-lysyl-1R,2S,4R,6R,7S-tricyclo[5.2.1.0$^{2.6}$]-3-aza-decan -4-carbonsäure-benzylester (Diastereomer F)

$N_{\alpha}$-(1-S-Carbethoxy-3-phenyl-propyl)-$N_{\varepsilon}$-benzyloxycarbonyl-S-lysyl-1R,2S,4S,6R,7S-tricyclo[5.2.1.0$^{2.6}$]-3-aza-decan-4-carbonsäure-benzylester (Diastereomer G)

$N_\alpha$-(1-S-Carbethoxy-3-phenyl-propyl)-$N_\varepsilon$ -benzyloxycarbonyl-S-lysyl-1S,2R,4R,6S,7R-tricyclo[5.2.1.$0^{2.6}$-3-aza-decan-4-carbonsäure-benzylester (Diastereomer H)

Nimmt man 1S,2S,4S,6R,7R-Tricyclo[5.2.1.$0^{2.6}$]-3-aza-decan-4-carbonsäure-benzylester, erhält man analog $N_\alpha$-(1-S-Carbethoxy-3-phenyl-propyl)-$N_\varepsilon$-benzyloxycarbonyl-S-lysyl-1S,2S,4S,6R,7R-tricyclo[5.2.1.$0^{2.6}$]-3-aza-decan-4-carbonsäure- benzylester (Diastereomer K).

**Beispiel 12**

Hydriert man die Diastereomeren E, F, G, H, K nach dem in Beispiel 10d beschriebenen Verfahren, so erhält man aus Diastereomer E

$N_\alpha$-(1-S-Carbethoxy-3-phenyl-propyl)-S-lysyl-1S,2R,4S,6S,7R-tricyclo[5.2.1.0.$^{2.6}$]-3-aza-decan-4-carbonsäure-dihydrochlorid (Diastereomer E')

$^1$H-NMR ($D_2O$): 0.9-3.0 (m, 18H), 3.0-4.9 (m, 14H), 7.2 (s, 5H),

aus Diastereomer F

$N_\alpha$-(1-S-Carbethoxy-3-phenyl-propyl)-S-lysyl-1R,2S,4R,6R,7S-tricyclo[5.2.1.$0^{2.6}$]-3-aza-decan-4-carbonsäure--dihydrochlorid (Diastereomer F')

$^1$H-NMR ($D_2O$): 1.0-3.2 (m, 18H); 3.2-5.1 (m, 14H); 7.1 (s, 5H),

aus Diastereomer G

$N_\alpha$-(1-S-Carbethoxy-3-phenyl-propyl)-S-lysyl-1R,2S,4S,6R,7S-tricyclo[5.2.1.$0^{2.6}$]-3-aza-decan-4-carbonsäure--dihydrochlorid (Diastereomer G')

$^1$H-NMR ($D_2O$): 1.0-3.3 (m, 20H); 3.4-5.0 (m, 12H); 7.2 (s, 5H),

aus Diastercomer H

$N_\alpha$-(1-S-Carbethoxy-3-phenyl-propyl)-S-lysyl-1S,2R,4R,6S,7R-tricyclo[5.2.1.$0^{2.6}$]-3-aza-decan-4-carbonsäure-dihydrochlorid (Diastereomer H')

$^1$H-NMR ($D_2O$): 0.9-3.0 (m, 18H); 3.0-4.9 (m, 14H); 7.2 (s, 5H)

aus Diastereomer K

$N_\alpha$-(1-S-Carbethoxy-3-phenyl-propyl)-S-lysyl-1S,2S,4S,6R,7R-tricyclo[5.2.1.$0^{2.6}$]-3-aza-decan-4-carbonsäure-dihydrochlorid (Diastereomer K')

$^1$H-NMR ($D_2O$): 1.0-3.1 (m, 18H); 3.0-4.8 (m, 14H); 7.2 (s, 5H)

**Beispeil 13**

$N_\alpha$-(1-S-Carboxy-3-phenyl-propyl)-S-lysyl-1R,2R,4S,6S,7S-tricyclo[5.2.1.$0^{2.6}$]-3-aza-decan-4-carbonsäure-hydrochlorid

0.5 g Ethylesterdihydrochlorid aus Beispiel 10d werden in 20 ml Dimethoxyethan suspendiert. Es wird wäßrige 4 n KOH zugegeben bis ein pH von 9-10 erreicht ist. Es wird eine halbe Stunde gerührt. Danach stellt man mit Salzsäure auf pH 4 ein, engt im Vakuum zur Trockene ein, nimmt den Rückstand in Essigester auf und filtriert. Die Essigesterlösung wiid eingeengt, der Rückstand wird mit Diisopropylether verrieben, wobei er fest wird.

Ausbeute 300 mg

$^1$H-NMR ($D_2O$): 0.9-2.9 (m, 15H); 3.0-4.9 (m, 12H); 7.2 (s, 5H)

**Beispiel 14**

Analog werden nach dem in Beispiel 13 beschriebenen Verfahren, ausgehend von den Diastereomeren E', F', G', H', K' die folgenden Dicarbonsäuren hergestellt:

$N_\alpha$-(1-S-Carboxy-3-phenyl-propyl)-S-lysyl-1S,2R,4S,6S,7R-tricyclo[5.2.1.$0^{2.6}$]-3-aza-decan-4-carbonsäure-hydrochlorid

$^1$H-NMR ($D_2O$): 1.0-3.0 (m, 15H); 3.0-5.0 (m, 12H); 7.2 (s, 5H)

$N_\alpha$-(1-S-Carboxy-3-phenyl-propyl)-S-lysyl-1R,2S,4R,6R,7S-tricyclo[5.2.1.0.$^{2.6}$]-3-aza-decan-4-carbonsäure-hydrochlorid

$N_\alpha$-(1-S-Carboxy-3-phenyl-propyl)-S-lysyl-1R,2S,4S,6R,7S-tricyclo[5.2.1.$0^{2.6}$]-3-aza-decan-4-carbonsäure-hydrochlorid

$^1$H-NMR ($D_2O$): 1.0-3.3 (m, 16H), 3.3-5.0 (m, 11H), 7.2 (s, 5H)

$N_\alpha$-(1-S-Carboxy-3-phenyl-propyl)-S-lysyl-1S,2R,4R,6S,7R-tricyclo[5.2.1.$0^{2.6}$]-3-aza-decan-4-carbonsäure-hydrochlorid

$N_\alpha$-(1-S-Carboxy-3-phenyl-propyl)-S-lysyl-1S,2S,4S,6R,7R,-tricyclo[5.2.1.$0^{2.6}$]-3-aza-decan-4-carbonsäure-hydrochlorid

$^1$H-NMR ($D_2O$): 1.0-3.1 (m, 15H); 3.1-4.9 (m, 12H); 7.2 (s, 5H)

**Beispiel 15**

S-Alanyl-1R,2R,4S,6S,7S-tricyclo[5.2.1.$0^{2.6}$]-3-aza-decan-4-carbonsäure-tert.butylester

a) 1:1-Gemisch 1R,2R,4S,6S,7S-Tricyclo[5.2.1.$0^{2.6}$]-3-aza-decan-4-carbonsäure-tert.butylester und entsprechendes Spiegelbildisomeres

2.5 g Aminosäure aus Beispiel 1d werden in 30 ml Dioxan mit 30 ml Isobutylen und 2.5 ml konzentrierter Schwefelsäure zur Reaktion gebracht. Nach 14 Stunden bei Raumtemperatur wird mit Natronlauge alkalisch gestellt, im Vakuum eingeengt, mit 20 ml Wasser versetzt und der Ester mit Methylenchlorid ausgeschüttelt. Nach Abdampfen des Methylenchlorids erhält man 2.0 g eines farblosen Öls.

$^1$H-NMR: 0.9 - 3.0 (m, 6H); 1.4 (s, 9H); 3.1-4.9 (m, 7H) (nach H/D-Austausch)

b) N-Benzyloxycarbonyl-S-Alanyl-1R,2R,4S,6S,7S-tricyclo-[5.2.1.$0^{2.6}$]-3-aza-decan-4-carbonsäure-tert.butylester

Zu einer Lösung von 1 g Z-Ala-OH in 10 ml Dimethylformamid werden 0.67 g 1-Hydroxy-benztriazol und 1,47 g des in Beispiel 15a hergestellten tert.Butylesters gegeben. Der pH-Wert wird mit N-Ethylmorpholin auf 8.0 eingestellt. Das Gemisch wird im Eisbad gekühlt und mit 1.05 g Dicyclohexylcarbodiimid versetzt. Man rührt 15 Stunden bei 20 - 25°C. Der ausgefallene Harnstoff wird abgesaugt, das Filtrat im Vakuum eingeengt und in Essigester aufgenommen. Die organische Phase wird nacheinander mit Kaliumhydrogensulfat-, Kaliumhydrogencarbonat- und Natriumchloridlösung gewaschen, getrocknet und eingedampft. Der Rückstand wird an Kieselgel mit Essigester/Cyclohexan 1:1 chromatographiert, um die Diastereomeren zu trennen. Ausbeute: 0.7 g N-Benzyloxycarbonyl-S-alanyl-1R,2R,4S,6S,7S-tricyclo[5.2.1.$0^{2.6}$]-3-aza-decan-4-carbonsäure-tert.butylester.

c) S-Alanyl-1R, 2R, 4S, 6S, 7S-tricyclo[5.2.1.$0^{2.6}$]-3-aza-decan-4-carbonsäure-tert.butylester

1.2 g des tert.Butylesters aus Beispiel 15b, werden in 20 ml Ethanol gelöst und mit 100 mg Pd/C (10%ig) bei Raumtemperatur und Normaldruck hydriert. Es wird vom Katalysator abgesaugt und der Rückstand im Vakuum eingeengt.

Ausbeute: 0.8 g farbloses Öl

$^1$H-NMR (nach H/D-Austausch): 0.9-3.1 (m, 6H); 1.2 (d, 3H); 1.4 (s, 9H); 3.1-5.0 (m, 8H)

**Beispiel 16**

N-(1-S,R-Carbethoxy-3-oxo-3-phenyl-propyl)-S-alanyl-1R,2R,4S,6S,7S-tricyclo[5.2.1.$0^{2.6}$]-3-aza-decan-4-carbonsäure-tert.butylester

Diese Verbindung wird aus der Verbindung von Beispiel 15b mit Benzoylacrylsäureethylester analog dem in Beispiel 10a beschriebenen Verfahren hergestellt.

**Beispiel 17**

N-(1-S,R-Carbethoxy-3-oxo-3-phenyl-propyl)-S-alanyl-1R,2R,4S,6S,7S-tricyclo[5.2.1.$0^{2.6}$]-3-aza-decan-4-carbonsäure-trifluoracetat.

0.5 g des in Beispiel 16 hergestellten tert.Butylesters werden in 5 ml Trifluoressigsäure gelöst und 30 min. bei Raumtemperatur gerührt. Danach wird die Trifluoressigsäure im Vakuum abgezogen und der Rückstand mit Diisopropyläther verrieben.

Ausbeute: 0.25 g fester Rückstand

$^1$H-NMR (nach H/D-Austausch): 1.0-3.2 (m, 12H); 3.3-4.9 (m, 13H); 7.2-8.2 (m, 5H).

**Beispiel 18**

N-(1-S,R-Carbethoxy-3-oxo-3-phenyl-propyl)-S-alanyl-1R, 2R, 4S, 6S, 7S-tricyclo[5.2.1.$0^{2.6}$]-3-aza-decan-4-carbonsäure-tert.butylester

5 mMol Acetophenon, 5 mMol Glyoxylsäureethylester und 5 mMol tert.Butylester des Beispiel 14 c) werden in 15 ml Eisessig 36 Stunden auf 45°C erhitzt. Nach dem Einengen im Vakuum wird mit Natriumbicarbonatlösung neutral gestellt und mit Essigester extrahiert. Die Essigesterphase wird eingeengt und an Kieselgel mit Essigester/Cyclohexan 1:1 als Elutionsmittel chromatographiert.

**Beispiel 19**

N-(1-S-Carbethoxy-3-phenyl-propyl)-S-alanyl-1R,2R,4S,6S,7S-tricyclo[5.2.1.0$^{2.6}$]-3-aza-decan-4-carbonsäure-tert.butylester

5 mMol tert.Butylester des Beispiel 15c werden in 15 ml wasserfreiem Ethanol gelöst. Man stellt die Lösung mit ethanolischem Kaliumhydroxid auf pH 7,0 und gibt 0,7 g pulverisiertes Molekularsieb (4Å) und anschließend 5 mMol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25°C wird die Lösung filtriert und das Lösungsmittel abdestilliert. Der Rückstand wird in Essigester/Wasser aufgenommen. Nach dem Eindampfen der Essigesterphasen wird der Rückstand an Kieselgel mit Essigester/Cyclohexan 1:4 chromatographiert.

$^1$H-NMR: 1.0-3.0 (m, 16H); 1.4 (s, 9H); 3.0-5.0 (m, 11 H); 7.2 (s, 5H) (nach H/D-Austausch)

**Beispiel 20**

N-(1-S-Carbethoxy-3-phenyl-propyl)-S-alanyl-1R,2R,4S,6S,7S-tricyclo[5.2.1.0$^{2.6}$]-3-aza-decan-4-carbonsäure--hydrochlorid

0.4 g des in Beispiel 19 hergestellten tert.Butylesters werden in 5 ml Trifluoressigsäure gelöst und 30 min bei Raumtemperatur gerührt. Danach wird die Trifluoressigsäure im Vakuum abgezogen. Der Rückstand wird in Wasser/Methanol gelöst, mit Acetat-beladenen Ionenaustauscher digeriert bis der pH etwa 5 beträgt. Der Ionenaustauscher wird abfiltriert und die Lösung mit ethanolischer Salzsäure auf pH 1 gebracht. Das Lösungsmittel wird im Vakuum abgezogen und der Rückstand mit Äther verrieben.

Ausbeute: 0.25 g

$R_f$: 0.42 ($SiO_2$; Methylenchlorid/Methanol 8:2)

**Beispiel 21**

O-Ethyl-S-tyrosinyl-1R,2R,4S,6S,7S-tricyclo[5.2.1.0$^{2.6}$]-3-aza-decan-4-carbonsäure-tert.butylester

a) N-Benzyloxycarbonyl-O-ethyl-S-tyrosinyl-1R,2R,4S,6S,7S-tricyclo[5.2.1.0$^{2.6}$]-3-aza-decan-4-carbonsäure-tert.butylester

Die Verbindung wird aus O-Ethyl-Z-tyrosin-OH und dem tert.Butylester, der in Beispiel 15a beschrieben ist, analog dem in Beispiel 15b beschriebenen Verfahren hergestellt. Die Diastereomeren werden über Kieselgel mit Cyclohexan/Essigester getrennt.

$^1$H-NMR (nach H/D-Austausch): 0.9 - 3.0 (m, 11H); 1.4 (s, 9H); 3.0-4.9 (m, 12H); 6.6-7.0 (m, 4 H); 7.2 (s, 5H)

b) O-Ethyl-S-tyrosinyl-1R,2R,4S,6S,7S-tricyclo[5.2.1.0$^{2.6}$]-3-aza-decan-4-carbonsäure-tert.butylester (Diastereomer 1).

Die Verbindung wird durch Hydrierung des tert.Butylesters des Beispiel 21a analog Beispiel 15c hergestellt.

$^1$H-NMR (Nach H/D-Austausch): 1.0-3.1 (m, 11H); 1.3 (s, 9H); 3.1-4.9 (m, 10H); 6.6-7.0 (m, 4H)

Analog dem in Beispiel 21 beschriebenen Verfahren werden aus den entsprechenden Ausgangsmaterialien die folgenden Verbindungen erhalten:

O-Ethyl-S-tyrosinyl-1S,2S,4S,6R,7R-tricyclo[5.2.1.0$^{2.6}$]-3-aza-decan-4-carbonsäure-tert.butylester (Diastereomer 2)

O-Ethyl-S-tyrosinyl-1R,2S,4S,6R,7S-tricyclo[5.2.1.0$^{2.6}$]-3-aza-decan-4-carbonsäure-tert.butylester (Diastereomer 3)

O-Ethyl-S-tyrosinyl-1S,2R,4S,6S,7R-tricyclo[5.2.1.0$^{2.6}$]-3-aza-decan-4-carbonsäure-tert.butylester (Diastereomer 4).

**Beispiel 22**

N-(1-S-Carbethoxy-3-oxo-3-phenyl-propyl)-[O-ethyl-S-tyrosinyl]-1R,2R,4S,6S,7S-tricyclo[5.2.1.0$^{2.6}$]-3-aza-decan-4-carbonsäure-tert.butylester (Diastereomer 1').

Man erhält diese Verbindung aus dem Diastereomer 1 aus Beispiel 21b und Benzoylacrylsäureethylester analog dem in Beispiel 10a beschriebenen Verfahren.

Analog diesem Verfahren werden aus den Diastereomeren 2, 3 und 4 die folgenden Verbindungen erhalten:

N-(1-S-Carbethoxy-3-oxo-3-phenyl-propyl)-[O-ethyl-S-tyrosinyl]- 1S,2S,4S,6R,7R-tricyclo[5.2.1.0$^{2.6}$]-3-aza-decan-4-carbonsäure-tert.butylester (Diastereomer 2').

N-(1-S-Carbethoxy-3-oxo-3-phenyl-propyl)-[O-ethyl-S-tyrosinyl]-1R,2S,4S,6R,7S-tricyclo[5.2.1.0$^{2.6}$]-3-aza-decan-4-carbonsäure-tert.butylester (Diastereomer 3').

N-(1-S-Carbethoxy-3-oxo-3-phenyl-propyl)-[O-ethyl-S-tyrosinyl]-1S,2R,4S,6S,7R-tricyclo[5.2.1.0$^{2.6}$]-3-aza-

decan-4-carbonsäure-tert.butylester (Diastereomer 4')

**Beispiel 23**

N-(1-S-Carbethoxy-3-oxo-3-phenyl-propyl)-[O-ethyl-S-tyrisinyl]-1R,2R,4S,6S,7S-tricyclo[5.2.1.$0^{2.6}$]-3-aza decan-4-carbonsäure-trifluoracetat

Diastereomer 1' des Beispiel 22 wird nach dem in Beispiel beschriebenen Verfahren umgesetzt.

$^{1}$H-NMR (nach H/D-Austausch): 1.0-3.1 (m, 16H); 3.1-4.9 (m, 13H); 6.6-7.0 (m, 4H); 7.2 (s, 5H)

Analog diesem Verfahren werden aus den Diastereomeren 2', 3' und 4' die folgenden Verbindungen erhalten:

N-(1-S-Carbethoxy-3-oxo-3-phenyl-propyl)-[O-ethyl-S-tyrosinyl]-1S,2S,4S,6R,7R-tricyclo[5.2.1.$0^{2.6}$]-3-aza-decan-4-carbonsäure-trifluoracetat

N-(1-S-Carbethoxy-3-oxo-3-phenyl-propyl)-[O-ethyl-S-tyrosinyl]-1R,2S,4S,6R,7S-tricyclo[5.2.1.$0^{2.6}$]-3-aza-decan-4-carbonsäure-trifluoracetat

N-(1-S-Carbethoxy-3-oxo-3-phenyl-propyl)-[O-ethyl-S-tyrosinyl]-1S,2R,4S,6S,7R-tricyclo[5.21.$0^{2.6}$]-3-aza-decan-4-carbonsäure-trifluoracetat

**Beispiel 24**

N-(1-S-Carbethoxy-3-phenyl-propyl)-[O-ethyl-S-tyrosinyl]-1R,2R,4S,6S,7S-tricyclo[5.2.1.$0^{2.6}$]-3-aza-decan-4 -carbonsäure-tert.butylester (Diastereomer 1)

Man erhält diese Verbindung aus der Verbindung des Beispiel 21b und 2-Keto-4-phenyl-buttersäureethylester analog nach dem in Beispiel 19 beschriebenen Verfahren.

$^{1}$H-NMR (nach H/D-Austausch): 1.0-3.0 (m, 18H); 1.4 (s, 9H); 3.1-5.0 (m, 13H); 6.6-7.0 (m, 4H); 7.2 (s, 5H)

Analog diesem Verfahren erhält man aus den Diastereomeren 2, 3, 4 des Beispiel 21 und 2-Keto-4-phenyl-buttersäureethylester die folgenden Verbindungen:

N-(1-S-Carbethoxy-3-phenyl-propyl)-[0-ethyl-S-tyrosinyl)-1S,2S,4S,6R,7R-tricyclo[5.2.1.$0^{2.6}$]-3-aza-decan-4-carbonsäure-tert.butylester (Diastereomer 2)

$^{1}$H-NMR (nach H/D-Austausch): 1.0-3.0 (m, 18H); 1.4 (s, 9H); 3.0-4.9 (m, 13H); 6.6-7.0 (m, 4H); 7.2 (s, 5H)

N-(1-S-Carbethoxy-3-phenyl-propyl)-[O-ethyl-S-tyrosinyl]-1R,2S,4S,6R,7S-tricyclo[5.2.1.$0^{2.6}$]-3-aza-decan-4-carbonsäure-tert.butylester (Diastereomer 3)

$^{1}$H-NMR (nach H/D-Austausch): 1.0-3.0 (m, 18H); 1.4 (s, 9H); 3.0-5.0 (m, 13H); 6.6-7.0 (m, 4H); 7.2 (s, 5H)

>N-(1-S-Carbethoxy-3-phenyl-propyl)-[O-ethyl-S-tyrosinyl]-1S,2R,4S,6S,7R-tricyclo[5.2.1.$0^{2.6}$]-3-aza-decan-4-carbonsäure-tert.butylester (Diastereomer 4)

$^{1}$H-NMR (nach H/D-Austausch): 0.9-3.1 (m, 18H); 1.4 (s, 9H); 3.2-4.9 (m, 13H); 6.6-7.0 (m, 4H); 7.2 (s, 5H)

**Beispiel 25**

N-(1-S-Carbethoxy-3-phenyl-propyl)-[O-ethyl-S-tyrosinyl]-1R,2R,4S,6S,7S-tricyclo[5.2.1.$0^{2.6}$]-3-aza-decan-4-carbonsäure-hydrochlorid

Durch Umsetzung des tert.Butylesters des Beispiel 24 mit Trifluoressigsäure analog dem in Beispiel 20 beschriebenen Verfahren wird die Carbonsäure erhalten.

$^{1}$H-NMR (nach H/D-Austausch): 1.0-3.1 (m, 18H); 3.1-5.0 (m, 13H); 6.6-7.0 (m, 4H); 7.2 (s, 5H)

Analog erhält man aus den Diastereomeren 2, 3, 4 des Beispiel 24 die folgenden Verbindungen:

N-(1-S-Carbethoxy-3-phenyl-propyl)-[O-ethyl-S-tyro-sinyl]-1S,2S,4S,6R,7R-tricyclo[5.2.1.$0^{2.6}$]-3-aza-decan-4-carbonsäure-hydrochlorid

$^{1}$H-NMR (nach H/D-Austausch): 0.9-3.0 (m, 18H), 3.0-4.9 (m, 13H); 6.6-7.0 (m, 4H); 7.2 (s, 5H)

N-(1-S-Carbethoxy-3-phenyl-propyl)-[O-ethyl-S-tyrosinyl]-1R,2S,4S,6R,7S-tricyclo[5.2.1.$0^{2.6}$]-3-aza-decan-4-carbonsäure-hydrochlorid

$^{1}$H-NMR (nach H/D-Austausch): 1.0-2.9 (m, 18H); 3.0-4.9 (m, 13H); 6.6-7.0 (m, 4H); 7.2 (s, 5H)

N-(1-S-Carbcthoxy-3-phenyl-propyl)-[O-ethyl-S-tyrosinyl]-1S,2R,4S,6S,7R-tricyclo[5.2.1.$0^{2.6}$]-3-aza-decan-4-carbonsäurehydrochlorid

$^{1}$H-NMR (nach H/D-Austausch): 1.0-3.1 (m, 18H); 3.1-5.0 (m, 13H); 6.6-7.0 (m, 4H); 7.2 (s, 5 H)

**Patentansprüche** für die Vertragsstaaten: BE, CH, DE, FR, GB, IT, LI, LU, NL, SE.

1. Verbindungen der Formel I,

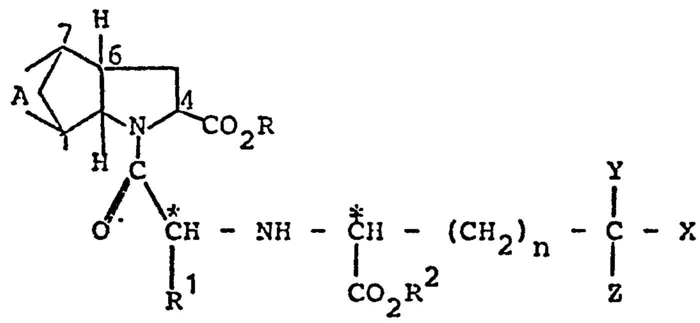

(I)

in welcher
n = 0 oder 1,
A = -CH=CH- oder $-CH_2-CH_2$
R = Wasserstoff, ($C_1$ bis $C_6$)-Alkyl oder Aralkyl mit 7 bis 9 C-Atomen,
$R^1$ = Wasseistoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)- Cycloalkyl-($_1$ bis $C_4$)-alkyl, ($C_6$ bis $C_4$)-Alkyl, ($C_1$ oder $C_2$)-Alkoxy oder Halogen subsituiert sein kann, ($C_6$-$C_{12}$)-Aryl-($C_1$ bis $C_4$)-alkyl oder ($C_7$-$C_{13}$)-Aroyl-($C_1$-$C_4$)-alkyl die beide wie vorstehend definiert, im Arylrest substituiert sein können, ein mono- bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer gegebenenfalls geschützten natürlich verkommenden Aminosäure,
$R^2$ = Wasserstoff, ($C_1$ bis $C_6$)-Alkyl, ($C_2$ bis $C_6$)-Alkenyl oder ($C_6$-$C_{12}$)-Aryl-($C_1$ bis $C_4$)-alkyl,
Y = Wasserstoff oder Hydroxy,
Z = Wasserstoff oder
Y und Z = zusammen Sauerstoff und
X = ($C_1$ bis $C_6$)-Alkyl, ($C_2$ bis $C_6$)-Alkenyl, ($C_5$ bis $C_9$)-Cycloalkyl, ($C_6$-$C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylendioxy, mono-, di- oder trisubstituiert sein kann, oder 3-Indolyl
-bedeuten,
sowie deren physiologisch unbedenkliche Salze.

2. Verbindungen der Formel I gemäß Anspruch 1, dadurch gekennzeichnet, daß die Wasserstoffatome an den Brückenkopf-C-Atomen 2 und 6 zueinander cis-konfiguriert sind, der Pyrrolidiering an den C-Atomen 2 und 6 endo- oder exo-ständig zum Bicyclus orientiert ist und die $-CO_2R$-Gruppe am C-Atom 4 cis- oder trans-ständig zum H-Atom an C-Atom 2 orientiert ist.

3. Verbindungen der Formel I gemäß einem der Ansprüche 1 und 2 dadurch gekennzeichnet, daß das C-Atom in Position 4 der Formel I sowie die mit einem Stern markierten C-Atome der Seitenkette jeweils S-Konfiguration aufweisen.

4. Verbindungen der Formel I gemäß einem der Ansprüche 1 bis 3, dadurch gekennzeichnet, daß
n = 1,
A = $CH_2-CH_2$
R = Wasserstoff oder ($C_1$ bis $C_4$)-Alkyl,
$R^1$ = Wasserstoff, ($C_1$ bis $C_3$)-Alkyl, ($C_2$ oder $C_3$)-Alkenyl, Benzyl, 4-Alkoxy-benzyl, Phenethyl, 4-Amino-butyl oder Benzoylmethyl oder eine Seitenkette einer gegebenenfalls geschützten natürlich vorkommenden Aminosäure
$R^2$ = Wasserstoff, ($C_1$ bis $C_4$)-Alkyl oder Benzyl,
X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro und/oder Methylendioxy mono- oder disubstituiert oder, im Falle von Methoxy, trisubstituiert sein kann, bedeuten.

5. Verbindungen der Formel I gemäß einem der Ansprüche 1 bis 4, dadurch gekennzeichnet, daß R = Waserstoff, $R^1$ = Methyl und X = Phenyl bedeuten.

6. Verbindungen der Formel I gemäß einem der Ansprüche 1 bis 5, dadurch gekennzeichnet, daß R = Wasserstoff, $R^1$ = Methyl, X = Phenyl und $R^2$ = Wasserstoff oder Ethyl bedeuden.

7. Verbindung der Formel I gemäß Anspruch 2, dadurch gekennzeichnet, daß die Carboxygruppe am C-Atom in Position 4 der Formel I cis- oder trans-ständig zum H-Atom an C-Atom 2 orientiert ist, der Pyrrolidinring exo- oder endo-ständig zum Bicyclus orientiert ist und das C-Atom 4 der Formel I sowie die mit einem Stern markierten C-Atome der Seitenkette jeweils S-Konfiguration aufweisen,
n = 1,
A = $CH_2-CH_2$

R = Wasserstoff
$R^1$ = Methyl,
$R^2$ = Ethyl,
Y und Z jeweils = Wasserstoff und
X = Phenyl bedeuten.

8. Verfahren zur Herstellung der Verbindungen der Formel I gemäß den Ansprüchen 1 bis 7, dadurch gekennzeichnet,

a) daß man eine Verbindung der Formel II

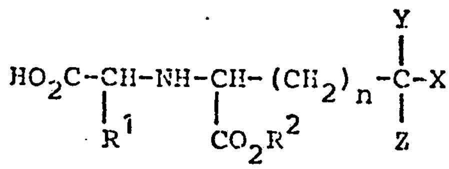

(II)

worin n, $R^1$ $R^2$, X, Y und Z die Bedeutung wie in Formel I haben mit einer Verbindung der Formel III,

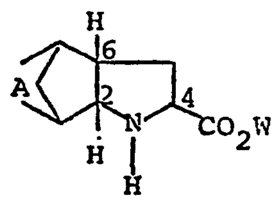

(III)

in welcher

W = Wasserstoff oder einen sauer oder basisch oder hydrogenolytisch abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV,

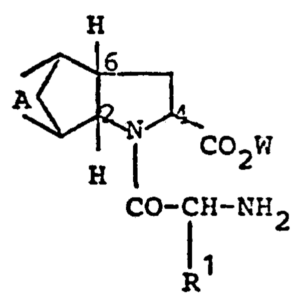

(IV)

in welcher $R^1$ die Bedeutung wie in Formel I und W die Bedeutung wie in Formel III besitzt, mit einer Verbindung der Formel V,

$$R^2O_2C-CH=CH-CO-X \qquad V$$

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindungen der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC-CO_2R^2 \qquad\qquad X-CO-CH_3$$

(VI) (VII)

worin X die Bedeutung wie in Formel I hat, umsetzt und anschließend gegebenenfalls W und/oder $R^2$ unter Bildung der freien carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

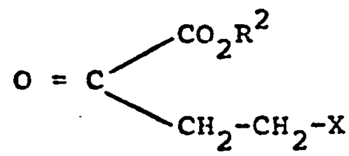

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert,

die nach a) bis d) erhaltenen Verbindungen der Formel I, in welcher R für Wasserstoff steht, gegebenfalls in Ester der Formel I, woring R ($C_1$ bis $C_5$)-Alkyl oder ($C_7$ bis $C_9$)-Aralkyl bedeutet, überführt und Verbindungen der Formel I gegebenenfalls in ihre physiologisch unbedenklichen Salze überführt.

9. Verbindung gemäß einem der Ansprüche 1 bis 7 zur Verwendung als Heilmittel.

10. Heilmittel, enthaltend eine Verbindung gemäß einem der Ansprüche 1 bis 7.

11. Verbindungen der Formel III,

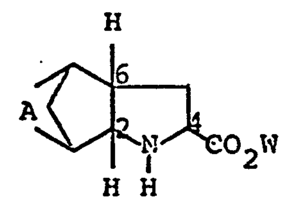

(III)

in der die H-Atome an den C-Atomen 2 und 6 zueinander cis-konfiguriert sind und die Gruppe $-CO_2W$ an C-Atom 4 cis- oder trans-ständig zum H-Atom an C-Atom 2 orientiert ist und der Pyrrolidinring exo- oder endo-ständig zum Bicyclus orientiert ist und worin W = Wasserstoff oder einen sauer, basisch oder hydrogenolytisch abspaltbaren Rest bedeutet.

12. Verfahren zur Herstellung von Verbindungen der Formel III gemäß Anspruch 11, dadurch gekennzeichnet, daß man

Verbindungen der Formel XI,

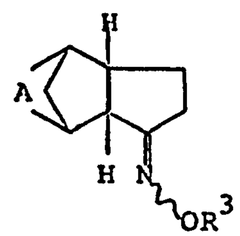

(XI)

in welcher A = CH=CH oder $CH_2$-$CH_2$, $R^3$ = Wasserstoff, Alkyl, Aryl, Aralkyl, $-SO_3H$, Benzolsulfonyl, p-Toluolsulfonyl, die H-Atome an den C-Atomen 2 und 6 cis zueinander konfiguriert sind, der Cyclopentanring endo-oder exo-ständig zum bicyclischen Ringsystem orientier ist, zu Verbindungen der Formel XII,

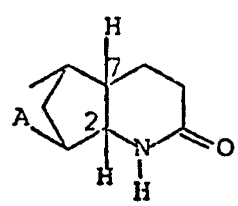

(XII)

in welcher A vorstehende Bedeutung hat, die H-Atome an den C-Atomen 2 und 7 cis-konfiguriert sind und der Laktamring exo- oder endo-ständig zum Bicyclus orientiert ist, umsetzt, diese zu Verbindungen der Formel XIII halogeniert,

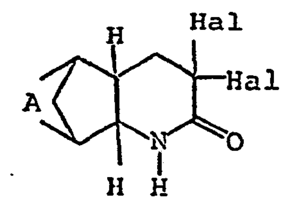

(XIII)

in welcher A obige Bedeutung hat und die Stereochemie der Formel XII entspricht, die Verbindungen der Formel XIII zu der Verbindung der Formel XIV katalytisch reduziert,

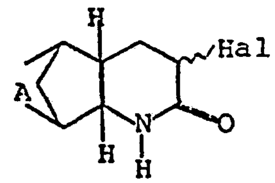

(XIV)

in welcher A und Hal die obengenannten Bedeutungen haben, wobei im Falle von A =(CH = CH) diese Doppelbindung vorübergehend als Cyclopentadienyleisen dicarbonyl-Komplex geschützt wird, die erhaltenen Verbindungen der Formel XIV in an sich bekannter Weise in Gegenwart einer Base zu einer Verbindung der Formel III mit W = Wasserstoff umgesetzt und diese gegebenenfalls verestert.

13. Verbindung gemäß einem der Ansprüche 1 bis 7 zur Verwendung als Heilmittel in Kombination mit einem Diuretikum

14. Heilmittel, enthaltend eine Verbindung gemäß einem der Ansprüche 1 bis 7 in Kombination mit einem Diuretikum.

**Patentansprüche** für den Vestragsstaat: AT.

1. Verfahren zur Herstellung der Verbindungen der Formel I

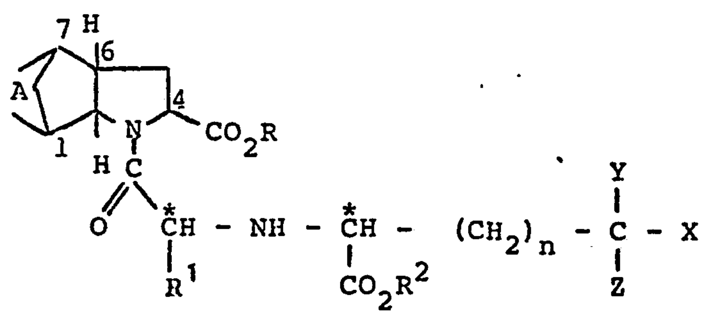

(I)

in welcher
n = 0 oder 1,
A = -CH=CH- oder $-CH_2-CH_2$
R = Wasserstoff, ($C_1$ bis $C_6$)-Alkyl oder Aralkyl mit 7 bis 9 C-Atomen,
$R^1$= Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{12}$)-Aryl oder teilhydriertes ($C_6$ bis $C_{12}$)-Aryl, das jeweils durch ($C_1$ bis $C_4$)-Alkyl, ($C_1$ oder $C_2$)-Alkoxy oder Halogen substituiert sein kann, ($C_6$ bis $C_{12}$)-Aryl-($C_1$ bis $C_4$)-alkyl oder ($C_7$ bis $C_{13}$)-Aroyl-($C_1$ bis $C_4$)alkyl die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer gegebenenfalls geschützten natürlich vorkommenden Aminosäure,
$R^2$= Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,
Y = Wasserstoff oder Hydroxy,
Z = Wasserstoff oder

Y und Z = zusammen Sauerstoff und
X = ($C_1$ bis $C_6$)-Alkyl ($C_2$ bis $C_6$)-Alkenyl, ($C_5$ bis $C_9$)-Cycloalkyl, ($C_6$ bis $C_{12}$)-Aryl, das durch ($C_1$ bis $C_4$)-Alkyl, ($C_1$ bis $C_4$)-Alkoxy Hydroxy, Halogen, Nitro, Amino, ($C_1$ bis $C_4$)-Alkylamino, Di-($C_1$ bis $C_4$)-alkyl-amino und/oder Methylendioxy mono-, di- oder trisubstituiert sein kann, oder 3-Indolyl
bedeuten,
sowie deren physiologisch unbedenklichen Salzen, dadurch gekennzeichnet,
a) daß man eine Verbindung der Formel II

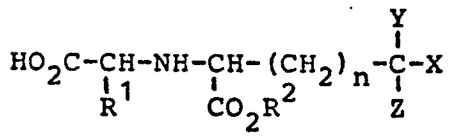

(II)

worin n, $R^1$, $R^2$, X, Y und Z die Bedeutung wie in Formel I haben mit einer Verbindung der Formel III,

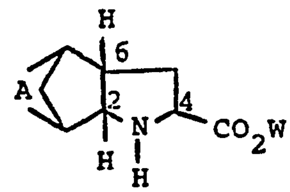

(III)

in welcher
W = Wasserstoff oder einen sauer oder basisch oder hydrogenolytisch abspaltbaren Rest bedeutet, umsetzt und anschließend gegebenenfalls W und/oder $R^2$ unter Bildung der freien Carboxygruppen abspaliet, oder
b) daß man zur Herstellung der Verbindungen der Formel I, in der Y und Z zusammen Sauerstoff bedeuten, $b_1$) ein Verbindung der Formel IV,

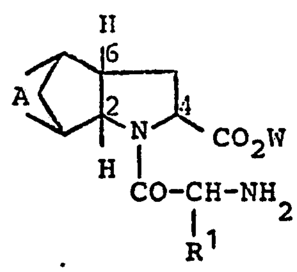

(IV)

in welcher $R^1$ die Bedeutung wie in Formel I und W die Bedeutung wie in Formel III besitzt, mit einer Verbindung der Formel V,

$$R^2O_2C-CH=CH-CO-X \qquad (V)$$

worin $R^2$ und X die Bedeutungen wie in Formel I haben, umsetzt und anschließend gegebenenfalls W und/oder $R^2$ unter Bildung der freien Carboxygruppen abspaltet, oder
$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC-CO_2R^2 \qquad\qquad X-CO-CH_3$$

(VI) (VII)

worin X die Bedeutung wie in Formel I hat, umsetzt und anschließend gegebenenfalls W und/oder $R^2$ unter Bildung der freien Carboxygruppen abspaltet, oder
c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

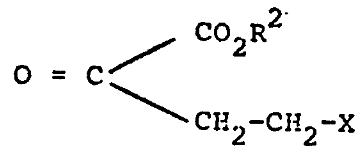 (VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert,

die nach a) bis d) erhaltenen Verbindungen der Formel I, in welcher R für Wasserstoff steht, gegebenenfalls in Ester der Formel I, worin R ($C_1$ bis $C_6$)-Alkyl oder $C_7$ bis $C_9$)-Aralkyl bedeutet, überführt und Verbindungen der Formel I gegebenenfalls in ihre physiologisch unbedenklichen Salze überführt.

2. Verfahren gemäß Anspruch 1 zur Herstellung von Verbindungen der Formel I, worin die Wasserstoffatome an den Brückenkopf-C-Atomen 2 und 6 zueinander cis-konfiguriert sind, der Pyrrolidinring an den C-Atomen 2 und 6 endo-oder exo-ständig zum Bicyclus orientiert ist und die $-CO_2R$-Gruppe am C-Atom 4 cis- oder trans-ständig zum H-Atom an C-Atom 2 orientiert ist.

3. Verfahren gemäß Anspruch 1 oder 2 zur Herstellung von Verbindungen der Formel I, worin das C-Atom in Position 4 der Formel I sowie die mit einem Stern markierten C-Atome der Seitenkette jeweils S-Konfiguration aufweisen.

4. Verfahren gemäß einem der Ansprüche 1 bis 3 zur Herstellung von Verbindungen der Formel I, worin

n = 1,

A = $CH_2$-$CH_2$

R = Wasserstoff oder ($C_1$ bis $C_4$)-Alkyl,

$R^1$ = Wasserstoff, ($C_1$-bis $C_3$)-Alkyl, ($C_2$ oder $C_3$)-Alkenyl, Benzyl, 4-Alkoxy-benzyl, Phenethyl, 4-Amino-butyl oder Benzoylmethyl oder eine Seitenkette einer gegebenenfalls geschützten natürlich vorkommenden Aminosäure

$R^2$ = Wasserstoff, ($C_1$ bis $C_4$)-Alkyl oder Benzyl,

X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro und/oder Methylendioxy mono- oder disubstituiert oder, im Falle von Methoxy, trisubstituiert sein kann,

bedeuten.

5. Verfahren gemäß einem der Ansprüche 1 bis 4 zur Herstellung von Verbindungen der Formel I, worin R = Wasserstoff, $R^1$ = Methyl und X = Phenyl bedeuten.

6. Verfahren gemäß einem der Ansprüche 1 bis 5 zur Herstellung von Verbindungen der Formel I, worin R = Wasserstoff, $R^1$ = Methyl, X = Phenyl und $R^2$ = Wasserstoff oder Ethyl bedeuten.

7. Verfahren gemäß Anspruch 2 zur Herstellung eine Verbindung der Formel I, worin die Carboxygruppe am C-Atom in Position 4 der Formel I cis- oder trans-ständig zum H-Atom an C-Atom 2 orientiert ist, der Pyrrolidinring exo-oder endo-ständig zum Bicyclus orientiert ist und das C-Atom 4 der Formel I sowie die mit einem Stern markierten C-Atome der Seitenkette jeweils S-Konfiguration aufweisen,

n = 1,

A = $CH_2$-$CH_2$

R = Wasserstoff

$R^1$ = Methyl,

$R^2$ = Ethyl,

Y und Z jeweils = Wasserstoff und

X = Phenyl bedeuten.

8. Verfahren zur Herstellung eines Heilmittels, enthaltend eine Verbindung der Formel I gemäß einem der Ansprüche 1 bis 7, dadurch gekennzeichnet, daß man diese in eine geeignete Darreichungsform bringt.

9. Verfahren zur Herstellung von Verbindungen der Formel III

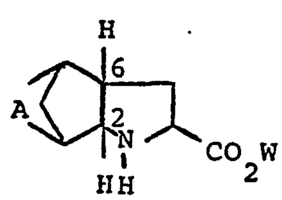 (III)

in der die H-Atome an den C-Atomen 2 und 6 zueinander cis-konfiguriert sind und die Gruppe $-CO_2W$ an C-Atom 4 cis- oder trans-ständig zum H-Atom an C-Atom 2 orientiert ist und der Pyrrolidinring exo- oder endo-ständig zum Bicyclus orientiert ist und worin W = Wasserstoff oder einen sauer, basisch oder hydrogenolytisch abspaltbaren Rest bedeutet, dadurch gekennzeichnet daß man Verbindungen der Formel XI,

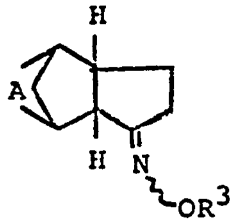

(XI)

in welcher A = CH=CH oder $CH_2$-$CH_2$, $R^3$ = Wasserstoff, Alkyl, Aryl, Aralkyl, -$SO_3H$, Benzolsulfonyl, p-Toluolsulfonyl, die H-Atome an den C-Atomen 2 bis 6 cis zueinander konfiguriert sind, der Cyclopentanring endo-oder exo-ständig zum bicyclischen Ringsystem orientiert ist, zu Verbindungen der Formel XII,

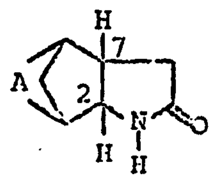

(XII)

in welcher A vorstehende Bedeutung hat, die H-Atome an den C-Atomen 2 und 7 cis-konfiguriert sind und der Laktamring exo- oder endo-ständig zum Bicyclus orientiert ist, umsetzt,

diese zu Verbindungen der Formel XIII halogeniert,

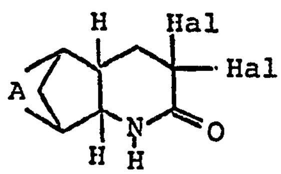

(XIII)

in welcher A obige Bedeutung hat und die Stereochemie der Formel XII entspricht, die Verbindungen der Formel XIII zu der Verbindung der Formel XIV katalytisch reduziert,

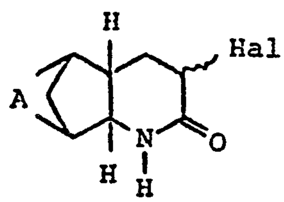

(XIV)

in welcher A und Hal die obengenannten Bedeutungen haben, wobei im Falle von A = (CH=CH) diese Doppelbindung vorübergehend als Cyclopentadienyleisen-dicarbonyl-Komplex geschützt wird, die erhaltenen Verbindungen der Formel XIV in an sich bekannter Weise in Gegenwart einer Base zu eincr Verbindung der Formel III mit W = Wasserstoff umgesetzt und diese gegebenenfalls verestert.

10. Verfahren zur Herstellung eines Heilmittel, enthaltend eine Verbindung der Formel I gemäß einem der Ansprüche 1 bis 7 in Kombiation mit einem Diuretikum, dadurch gekennzeichnet, daß man diese in eine geeignete Darreichungsform bringt.

11. Verfahren gemäß einem der Ansprüche 1 bis 7 zur Herstellung einer Verbindung der Formel I zur Verwendung als Heilmittel.

**Claims** for the Contracting States: BE, CH, DE, FR, GB, IT, LI, LU, NL, SE.

1. Compounds of the formula I

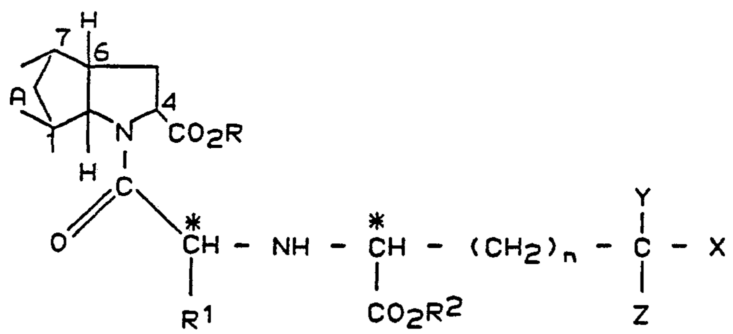

(I)

in wich
n denotes 0 or 1,
A denotes -CH = CH- or $-CH_2-CH_2-$,
R denotes hydrogen, ($C_1$ to $C_6$)-alkyl or aralkyl with 7 to 9 carbon atoms,
$R^1$ denotes hydrogen, or ($C_1$ to $C_6$)-alkyl, which can be optionally substituted by amino, ($C_1$ to $C_4$)-acylamino or benzoylamino, or ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $C_2$)-alkoxy or halogen, or ($C_6$ to $C_{12}$)-aryl-($C_1$ to $C_4$)-alkyl or ($C_7$ to $C_{13}$)-aroyl-($C_1$ to $C_4$)-alkyl, both of which can be substituted in the aryl radical as defined above, or a monocyclic or bicyclic heterocyclene radical with 5 to 7 or 8 to 10 ring atoms, 1 or 2 ring atoms of which are sulfur or oxygen atoms and/or 1 to 4 ring atoms of which are nitrogen atoms, or a side chain of a naturally occurring aminoacid, which may be protected,
$R^2$ denotes hydrogen, ($C_1$ to $C_6$)-alkyl, ($C_2$ to $C_6$)-alkenyl or ($C_2$ to $C_6$)-aryl-($C_1$ to $C_4$)-alkyl,
Y denotes hydrogen or hydroxyl and
Z denotes hydrogen, or
Y and Z together denote oxygen, and
X denotes ($C_1$ to $C_6$)-alkyl, ($C_2$ to $C_6$)-alkenyl, ($C_5$ to $C_9$)-cycloalkyl, ($C_6$ to $C_{12}$)-aryl, which can be mono-, di- or tri-substituted by ($C_1$ to $C_4$)-alkyl, ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino and/or methylenedioxy, or 3-indolyl, and physiologically acceptable salts thereof.

2. Compounds of the formula I according to claim 1, characterized in that the hydrogen atoms on the brige head C-2 and C-6 are in the cis-configuration relative to one another, the pyrrolidine ring on C-2 and C-6 is orientated in the endo- or exo-position relative to the bicyclic radical and the $-CO_2R$ group on C-4 is orientated in the cis- or trans-position relative to the H atom on C-2.

3. Compounds of the formula I according to claim 1 or 2, characterized in that the carbon atom in position 4 in the formula I and the carbon atoms labeled with an asterisk in the side chain each have the S-configuration.

4. Compounds of the formula I according to any of claims 1 to 3, characterized in that
n denotes 1,
A denotes $CH_2$-$CH_2$,
R denotes hydrogen or ($C_1$ to $C_4$)-alkyl,
$R^1$ denotes hydrogen, ($C_1$ to $C_3$)-alkyl, ($C_2$ to $C_3$)-alkenyl, benzyl, 4-alkoxybenzyl, phenethyl, 4-aminobutyl or benzoylmethyl or a side chain of a naturally occuring aminoacid, which be protected,
$R^2$ denotes hydrogen, ($C_1$ to $C_4$)-alkyl or benzyl, X denotes phenyl, which can be mono- or di-substituted by ($C_1$ to $C_2$)-alkyl, ($C_1$ to $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy or trisubstituted by methoxy.

5. Compounds of the formula I according to anyl of claims 1 to 4, characterized in that R denotes hydrogen, $R^1$ denotes methyl and X denotes phenyl.

6. Compounds of the formula I according to any of claims 1 to 5, characterized in that R denotes hydrogen, $R^1$ denotes methyl, X denotes phenyl and $R^2$ denotes hydrogen or ethyl.

7. Compounds of the formula I according to claim 2, characterized in that the carboxyl group on the carbon atom in position 4 of the formula I is orientated in the cis- or trans-position relative to the H atom on C-2, the pyrrolidine ring is orientated in the exo- or endo-position relative to the bicyclic radical, C-4 in the formula I and the carbon atoms labeled with an asterisk in the side chain each have the S-configuration,
n denotes 1,
A denotes $CH_2$-$CH_2$,
R denotes hydrogen,
$R^1$ denotes methyl,
$R^2$ denotes ethyl,
Y and Z each denote hydrogen and
X denotes phenyl.

8. A process for the preparation of compounds of the formula I according to any of claims 1 to 7, which comprises

a) reacting a compound of the formula II

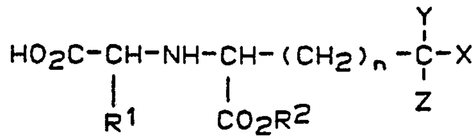

(II)

in which n, $R^1$, $R^2$, X, Y and Z have the meaning as in formula I, whith a compound of the formula III

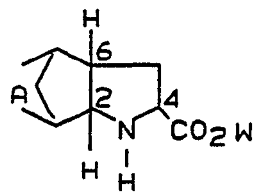

(III)

in which

W denotes hydrogen or a radical which can be split off under acid, basic or hydrogenolytic conditions, and, where relevant, subsequently splitting off W and/or $R^2$ to form free carboxyl groups, or

b) to prepare compounds of the formula I in which y and Z together denote oxygen,

$b_1$) reacting a compound of the formula IV

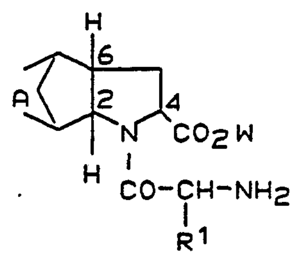

(IV)

in which $R^1$ has the meaning as in formula I and W has the meaning as in formula III, with a compound of the formula V

$$R^2O_2C-CH=CH-CO-X \quad (V)$$

in which $R^2$ and X have the meanings as in formula I, and, where relevant, subsequently splitting off W and/or $R^2$ ot form free carboxyl groups, or

$b_2$) reacting a compound of the formula IV mentioned under $b_1$) with a compound of the general formula VI, in which $R^2$ has the meaning as in formula I, and with a compound of the general formula VII

$$OHC-CO_2R^2 \quad (VI) \qquad X-CO-CH_3 \quad (VII)$$

in which X has the meaning as in formula I, and, where relevant, subsequently splitting off W and/or $R^2$ to form free carboxyl groups or

c) to prepare compounds of the formula I in which Y and Z each denote hydrogen, reacting a compound of the formula IV mentioned under $b_1$) with a compound of the formula VIII

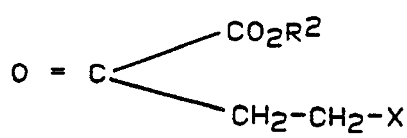

(VIII)

in which $R^2$ and X have meanings as in formula I, reducing the resulting Schiff's base and, where relevant, subseqently splitting off W and/or $R^2$ to form free carboxyl groups, or

d) to prepare compounds of the formula I in which Y denotes hydroxyl and Z denotes hydrogen, reducing a compound of the formula I in which Y and Z together denote oxygen with a complex boranate or a borane-amine complex, and, if desired, converting compounds of the formula I obtained according to a) to d), in which R represents hydrogen, into esters of the formula I in which R denotes ($C_1$ to $C_6$)-alkyl or ($C_7$ to $C_9$)-aralkyl, and optionally converting compounds of the formula I into their physiologically acceptable salts.

9. A compound according to any of claims 1 to 7 for the use as a medicine.

10. A pharmaceutical composition containing a compound according to any of claims 1 to 7.

11. Compounds of the formula III

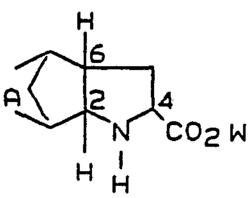

(III)

in which the H atoms on C-2 and C-6 are in the cis-configuration relative to one another, the group $-CO_2W$ on C-4 is orientated in the cis- or trans-position relative to the H atom on C-2 and the pyrrolidine ring is orientated in the exo- or endo-position relative to the bicyclic radical, and in which W denotes hydrogen or a radical which can be split off under acid, basic or hydrogenolytic conditions.

12. A process for the preparation of compounds of the formula III according to claim 11, which comprises reacting compounds of the formula XI

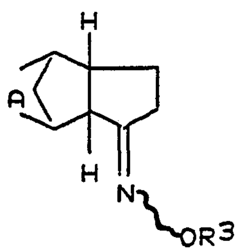

(XI)

in which A denotes $CH=CH$ or $CH_2$-$CH_2$, $R^3$ denotes hydrogen, alkyl, aryl, aralkyl, $-SO_3H$, benzenesulfonyl or p-toluenesulfonyl, the H atoms on C-2 and C-6 are in the cis-configuration relative to one another and the cyclopentane ring is orientated in the endo- or exo-position relative to the bicyclic ring system, to give compounds of the formula XII

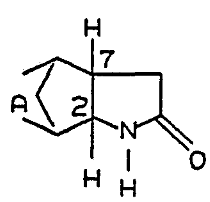

(XII)

in which A has the above meaning, the H atoms on C-2 and C-7 are in the cis-configuration and the lactam ring is orientated in the exo- or endo-position relative to the bicyclic radical, halogenating these compounds to give compounds of the formula XIII

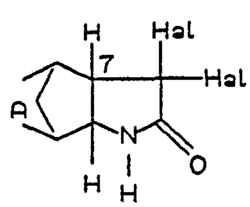

(XIII)

in which A has the above meaning and the stereochemistry corresponds to the formula XII, catalytically reducing the compounds of the formula XIII to give a compound of the formula XIV

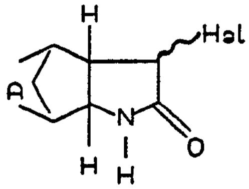

(XIV)

in which A and Hal have the abovementioned meanings and, if A denotes CH=CH, this double bond is transiently protected as a cyclopentadienyl-iron dicarbonyl complex, reacting the resulting compounds of the formula XIV in the presence of a base in a manner which is known per se to give a compound of the formula III in which W denotes hydrogen and, if desired, esterifying this compound.

13. A compound according to any of claims 1 to 7, in combination with a diuretic, for the use as a medicine.

14. A pharmaceutical composition containing a compound according to any of claims 1 to 7 in combination with a diuretic.

**Claims** for the Contracting State: AT

1. A process for the preparation of compounds of the formula I

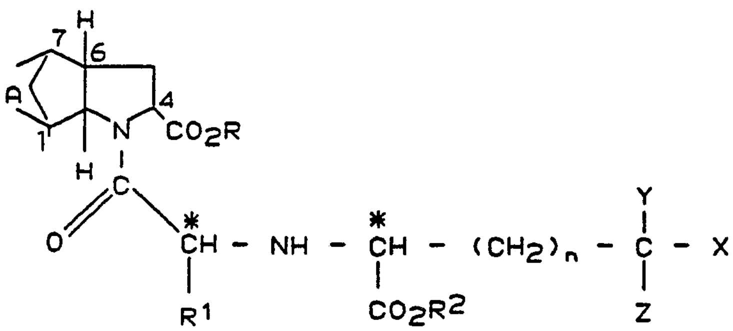

(I)

in which
n denotes 0 or 1,
A denotes $-CH=CH-$ or $-CH_2-CH_2-$,
R denotes hydrogen, ($C_1$ to $C_6$)-alkyl or aralkyl with 7 to 9 carbon atoms,
$R^1$ denotes hydrogen, or ($C_1$ to $C_6$)-alkyl, which can be optionally substituted by amino, ($C_1$ to $C_4$)-acylamino or benzoylamino, or ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{12}$)-aryl or partially hydrogenated ($C_6$ to $C_{12}$)-aryl, each of which can be substituted by ($C_1$ to $C_4$)-alkyl, ($C_1$ or $C_2$)-alkoxy or halogen, or ($C_6$ to $C_{12}$)-aryl-($C_1$ to $C_4$)-alkyl or ($C_7$ to $C_{13}$)-aroyl-($C_1$ to $C_4$)-alkyl, both of which can be substituted in the aryl radical as defined above, or a monocyclic or bicyclic heterocyclene radical with 5 to 7 or 8 to 10 ring atoms, 1 or 2 ring atoms of which are sulfur or oxygen atoms and/or 1 to 4 ring atoms of which are nitrogen atoms, or a side chain of a naturally occurring aminoacid, which may be protected,
$R^2$ denotes hydrogen, ($C_1$ to $C_6$)-alkyl, ($C_2$ to $C_6$)-alkenyl or ($C_2$ to $C_6$)-aryl-($C_1$ to $C_4$)-alkyl,
Y denotes hydrogen or hydroxyl and
Z denotes hydrogen, or
Y and Z together denote oxygen, and
X denotes ($C_1$ to $C_6$)-alkyl, ($C_2$ to $C_6$)-alkenyl, ($C_5$ to $C_9$)-cycloalkyl, ($C_6$ to $C_{12}$)-aryl, which can be mono-, di- or tri-substituted by ($C_1$ to $C_4$)-alkyl, ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino and/or methylenedioxy, or 3-indolyl, and physiologically acceptable salts thereof, which comprises

a) reacting a compound of the formula II

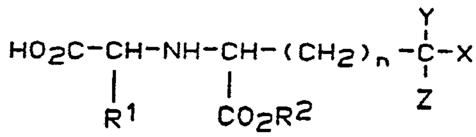

(II)

in which n, $R^1$, $R^2$, X, Y and Z have the meaning as in formula I, whith a compound of the formula III

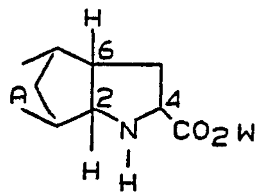

(III)

in which

W denotes hydrogen or a radical which can be split off under acid, basic or hydrogenolytic conditions, and, where relevant, subsequently splitting off W and/or $R^2$ to form free carboxyl groups, or

b) to prepare compounds of the formula I in which Y and Z together denote oxygen,

$b_1$ reacting a compound of the formula IV

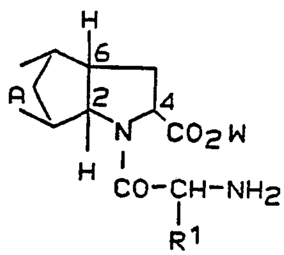

(IV)

in which $R^1$ has the meaning as in formula I and W has the meaning as in formula III, with a compound of the formula V

$$R^2O_2C\text{-}CH{=}CH\text{-}CO\text{-}X \qquad (V)$$

in which $R^2$ and X have the meanings as in formula I, and, where relevant, subseguently splitting off W and/or $R^2$ to form free carboxyl groups, or

$b_2$) reacting a compound of the formula IV mentioned under $b_1$) with a compound of the general formula VI, in which $R^2$ has the meaning as in formula I, and with a compound of the general formula VII

$$OHC\text{-}CO_2R^2 \qquad X\text{-}CO\text{-}CH_3$$

(VI) (VII)

in which X has the meaning as in formula I, and, where relevant, subsequently splitting off W and/or $R^2$ to form free carboxyl groups or

c) to prepare compounds of the formula I in which Y and Z each denote hydrogen, reacting a compound of the formula IV mentioned under $b_1$) with a compound of the formula VIII

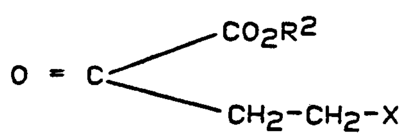

(VIII)

in which $R^2$ and X have meanings as in formula I, reducing the resulting Schiff's base and, where relevant, subsequently splitting off W and/or $R^2$ to form free carboxyl groups, or

d) to prepare compounds of the formula I in which Y denotes hydroxyl and Z denotes hydrogen, reducing a compound of the formula I in which Y and Z together denote oxygen with a complex boranate or a borane-amine complex, and, if desired, converting compounds of the formula I obtained according to a) to d), in which R represents hydrogen, into esters of the formula I in which R denotes ($C_1$ to $C_6$)-alkyl or ($C_7$ to $C_9$)-aralkyl, and optionally converting compounds of the formula I into their physiologically acceptable salts.

2. A process according to claim 1, for the preparation of compounds of the formula I, in which the hydrogen

atoms on the brige head C-2 and C-6 are in the cis-configuration relative to one another, the pyrrolidine ring on C-2 and C-6 is orientated in the endo- or exo-position relative to the bicyclic radical and the $-CO_2R$ group on C-4 is orientated in the cis- or trans-position relative to the H atom on C-2.

3. A process according to claim 1 or 2, for the preparation of compounds of the formula *I*, in which the carbon atom in position 4 in the formula I and the carbon atoms labeled with an asterisk in the side chain each have the S-configuration.

4. A process according to any of claims 1 to 3, for the preparation of compounds of the formula I, in which
n denotes 1,
A denotes $CH_2$-$CH_2$,
R denotes hydrogen or ($C_1$ to $C_4$)-alkyl,
$R^1$ denotes hydrogen, ($C_1$ to $C_3$)-alkyl, ($C_2$ to $C_3$)-alkenyl, benzyl, 4-alkoxybenzyl, phenethyl, 4-aminobutyl or benzoylmethyl or a side chain of a naturally occuring aminoacid, which be protected,
$R^2$ denotes hydrogen, ($C_1$ to $C_4$)-alkyl or benzyl, X denotes phenyl, which can be mono- or di-substituted by ($C_1$ to $C_2$)-alkyl, ($C_1$ to $C_2$)-alkoxy, hydroxyl, fluorine, chlorine, bromine, amino, ($C_1$ to $C_4$)-alkylamino, di-($C_1$ to $C_4$)-alkylamino, nitro and/or methylenedioxy or trisubstituted by methoxy.

5. A process according to anyl of claims 1 to 4, for the preparation of compounds of the formula I, in which R denotes hydrogen, $R^1$ denotes methyl and X denotes phenyl.

6. A process according to any of claims 1 to 5, for the preparation of compounds of the formula I, in which R denotes hydrogen, $R^1$ denotes methyl, X denotes phenyl and $R^2$ denotes hydrogen or ethyl.

7. A process according to claim 2, preparation of compounds of the formula I, in which the carboxyl group on the carbon atom in position 4 of the formula I is orientated in the cis- or trans-position relative to the H atom on C-2, the pyrrolidine ring is orientated in the exo- or endo-position relative to the bicyclic radical, C-4 in the formula I and the carbon atoms labeled with an asterisk in the side chain each have the S-configuration,
n denotes 1,
A denotes $CH_2$-$CH_2$,
R denotes hydrogen,
$R^1$ denotes methyl,
$R^2$ denotes ethyl,
Y and Z each denote hydrogen and
X denotes phenyl.

8. A process for the preparation of compounds of the formula III

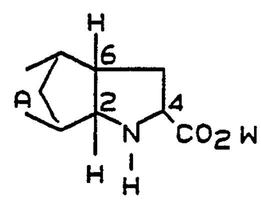

(III)

in which the H atoms on C-2 and C-6 are in the cis-configuration relative to one another, the group $-CO_2W$ on C-4 is orientated in the cis- or trans-position relative to the H atom on C-2 and the pyrrolidine ring is orientated in the exo- or endo-position relative to the bicycloc radical, and in which W denotes hydrogen or a radical which can be split off under acid, basic or hydrogenolytic conditions, which comprises reacting compounds of the formula XI

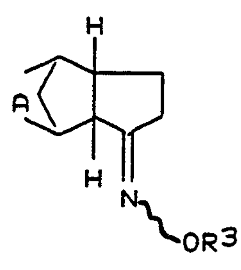

(XI)

in which A denotes CH=CH or $CH_2$-$CH_2$, $R^3$ denotes hydrogen, alkyl, aryl, aralkyl, $-SO_3H$, benzenesulfonyl or p-toluenesulfonyl, the H atoms on C-2 and C-6 are in the cis-configuration relative to one another and the cyclopentane ring is orientated in the endo- or exo-position relative to the bicyclic ring system, to give compounds of the formula XII

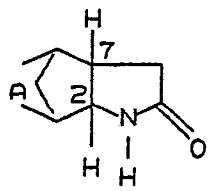

(XII)

in which A has the above meaning, the H atoms on C-2 and C-7 are in the cis-configuration and the lactam ring is orientated in the exo- or endo-position relative to the bicyclic radical, halogenating these compounds to give compounds of the formula XIII

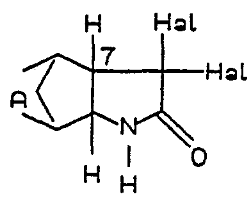

(XIII)

in which A has the above meaning and the stereochemistry corresponds to the formula XII, catalytically reducing the compounds of the formula XIII to give a compound of the formula XIV

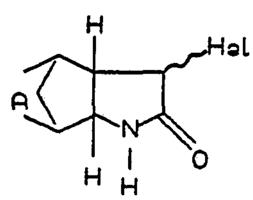

(XIV)

in which A and Hal have the above mentioned meanings and, if A denotes CH=CH, this double bond is transiently protected as a cyclopentadienyl-iron dicarbonyl complex, reacting the resulting compounds of the formula XIV in the presence of a base in a manner which is known per se to give a compound of the formula III in which W denotes hydrogen and, if desired, esterifying this compound.

9. A process for the preparation of a pharmaceutical composition, containing a compound of the formula according to any of claims 1 to 7 in combination with a diuretic, characterized in that the compound is converted into a suitable form for administration.

10. A process according to any of claims 1 to 7 for the preparation of a compound of the formula I for the use as a medicament.

11. A process according to any of claims 1 to 7 for the preparation of a compound of the formula I for the use as a medicament.

**Revendications** pour les Etats Contractants: BE, CH, DE, FR, GB, IT, LI, LU, NL, SE.

1. Composés de formule I

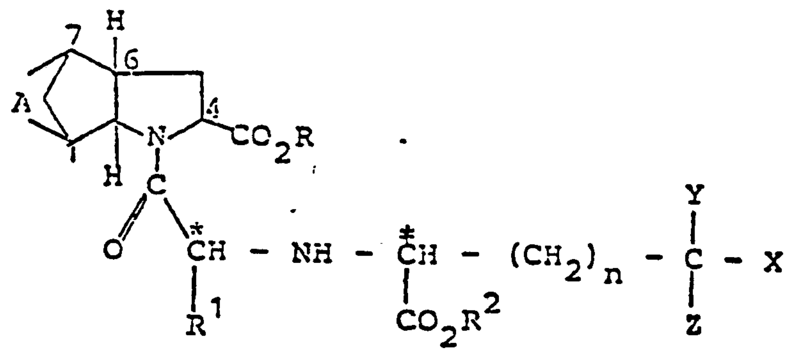

(I)

dans laquelle,
n est 0 ou 1,
A est -CH=CH- ou $-CH_2-CH_2-$,
R représente l'hydrogène, un groupe alcoyle en $C_1$-$C_6$ ou aralcoyle ayant de 7 à 9 atomes de carbone,
$R^1$ représente l'hydrogène ou un gorupe alcoyle en $C_1$-$C_6$ qui peut être éventuellement substitué par un groupe amino, acylamino en $C_1$-$C_4$ ou benzoylamino; un groupe alcényle en $C_2$-$C_6$ cycloalcoyle en $C_5$-$C_9$ cycloalcényle en $C_5$-$C_9$ cycloalcoyl ($C_5$-$C_7$)-alcoyle en $C_1$-$C_4$, aryle en $C_6$-$C_1$ ou aryle en $C_6$-$C_{12}$ partiellement hydrogéné, qui peuvent être chacun éventuellement substitués par un groupe alcoyle en $C_1$-$C_4$, alcoxy en $C_1$ ou $C_2$ ou par un halogène; un groupe aryl ($C_6$-$C_{12}$) alcoyle en $C_1$-$C_4$ ou aroyl ($C_7$-$C_{13}$)-alcoyle en $C_1$-$C_4$ qui peuvent être tous les deux substitués sur le groupe aryle tel que défini précédemment; un groupe hétérocyclique mono ou bicyclique ayant de 5 à 7 ou de 8 à 10 atomes dans le noyau, dont 1 à 2 atomes dans le noyau représentent des atomes de soufre ou d'oxygène et/ou dont 1 à 4 atomes dans le noyau représentent des atomes d'azote; ou une chaîne latérale d'un acide aminé naturel éventuellement protégé.
$R^2$ représente l'hydrogène, un groupe alcoyle en $C_1$-$C_6$, alcényle en $C_2$-$C_6$ ou aryl ($C_6$-$C_{12}$)-alcoyle en $C_1$-$C_4$,
Y représente l'hydrogène ou un groupe hydroxy,
Z représente l'hydrogène ou
Y et Z représentent ensemble l'oxygène et
X représente un groupe alcoyle en $C_1$-$C_6$, alcényle en $C_2$-$C_6$, cycloalcoyle en $C_5$-$C_9$ aryle en $C_6$-$C_{12}$, qui peut être mono-di- ou tri-substitué par un groupe alcoyle en $C_1$-$C_4$, alcoxy en $C_1$-$C_4$, hydroxy, un halogène, un groupe nitro, amino, alcoylamino en $C_1$-$C_4$, dialcoyl ($C_1$-$C_4$)-amino et/ou méthylène-dioxy; ou un groupe 3-indo-lyle,
et leurs sels physiologiquement acceptables.

2. Des composés de formule I selon la revendication 1, dont les atomes d'hydrogène sur les atomes de carbone, têtes de pont, 2 et 6, sont en configuration cis l'un par rapport à l'autre, le noyau pyrrolidine sur les atomes de carbone 2 et 6 a une orientation endo ou exo par rapport au composé bicyclique et le groupe $-CO_2R$ sur l'atome de carbone 4 a une orientation cis ou trans par rapport à l'atome d'hydrogène sur l'atome de carbone 2.

3. Des composés de formule I
1 ou 2, dont l'atome de carbone en position 4 dans la formule 1 ainsi que les atomes de carbone marqués d'un astérisque dans la chaîne latérale montrent tous une configuration S.

4. Des composés de formule I selon l'une quelconque des revendications 1 à 3 dans lesquels
n est 1
A est $-CH_2-CH_2-$
R représente l'hydrogène ou un groupe alcoyle en $C_1$-$C_4$,
$R^1$ représente l'hydrogène, un groupe alcoyle en $C_1$-$C_3$, alcényle en $C_2$ ou $C_3$, benzyle, 4-alcoxy-benzyle, phénéthyle, 4-amino-butyle ou benzoylméthyle ou une chaîne latérale d'un acide aminé naturel, éventuellement protégé,
$R^2$ représente l'hydrogène, un groupe alcoyle en $C_1$-$C_4$ ou benzyle,
X représente un groupe phényl qui peut être mono- ou disubstitué par un groupe alcoyle en $C_1$ ou $C_2$, alcoxy en $C_1$ ou $C_2$, hydroxy, le fluor, le chlore, le brome, un groupe amino, alcoylamino en $C_1$-$C_4$ di-alcoyl ($C_1$-$C_4$)-amino, nitro et/ou méthylène dioxy ou qui peut, dans le cas d'un groupe méthoxy, être trisubstitué par ces mêmes groupes.

5. Des composés de formule I
1 à 4, dans lesquels R représente l'hydrogène, $R^1$ représente un groupe méthyle et X représente un groupe phényle.

6. Des composés de formule I
1 à 5, dans lesquels R représente l'hydrogène, $R^1$ représente un groupe méthyle, X représente un groupe phényle et $R^2$ représente l'hydrogène ou un groupe éthyle.

7. Des composés de formule I selon la revendication 2, dans lesquels le groupe carboxy sur l'atome de carbone en position 4 de la formule I a une orientation cis ou trans par rapport à l'atome d'hydrogène sur l'atome de carbone 2, le noyau pyrrolidine a une orientation exo ou endo par rapport au système bicyclique et

l'atome de carbone 4 de la formule I ainsi que les atomes de carbone marqués d'un astérisque dans la chaîne latérale ont chacun une configuration S,
n est 1,
a est $-CH_2-CH_2-$
R représente l'hydrogène,
$R^1$ représente un groupe méthyle,
$R^2$ représente un groupe éthyle,
Y et Z représentent chacun l'hydrogène et
X représente un groupe phényle.

8. Un procédé pour la préparation des composés de formule I selon lequel:
a) on fait réagir un composé de formule II

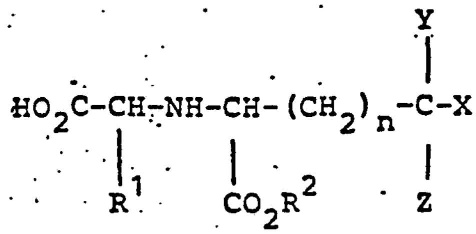

dans laquelle n', $R^1$, $R^2$, X, Y et Z ont les significations indiquées pour la formule I avec un composé de formule III

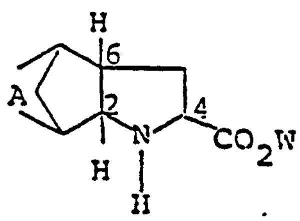

(III)

W représente l'hydrogène ou un groupe éliminable par un acide ou une base ou par hydrogénolyse, puis on élimine éventuellement W et/ou $R^2$ en formant les groupes carboxy libres, ou
b) pour la préparation des composés de formule I, dans lesquels Y et Z représentent ensemble l'oxygène,
$b_1$) on fait réagir un composé de formule IV

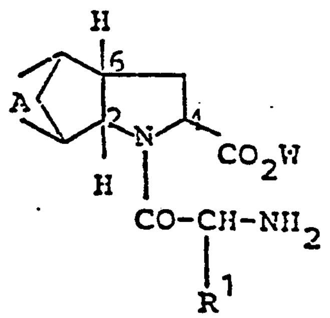

(IV)

dans laquelle $R^1$ a la signification indiquée pour la formule I et W a la signification indiquée pour la formule III, avec un composé de formule V

$$R^2O_2C-CH=CH-CO-X \qquad V$$

dans laquelle $R^2$, et X ont les significations indiquées pour la formule I, et éventuellement on élimine W et/ou $R^2$ en formant les groupes carboxy libres, ou
$b_2$) un composé de formule IV indiqué en $b_1$), avec un composé de formule générale VI, dans lequel $R^2$ a la signification indiquée pour la formule I, et avec un composé de formule générale VII

$$OHC-CO_2R^2 \quad (VI) \qquad X-CO-CH_3 \quad (VII)$$

dans laquelle X a la signification indiquée pour la formule I, et éventuellement on élimine W et/ou $R^2$ en formant les groupes carboxy libres, ou

c) pour préparer des composés de formule I, dans lesquels Y et Z représentent chacun l'hydrogène, on fait réagir un composé de formule IV indiquée en $b_1$) avec un composé de formule VIII

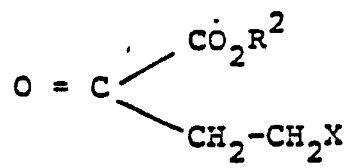

VIII

dans laquelle $R^2$ et X ont les significations indiquées pour la formule I, on réduit les bases de Schiff obtenues et éventuellement on élimine W et/ou $R^2$ en formant les groupes carboxy libres, ou

d) pour préparer des composés de formule I, dans lesquels Y représente un groupe hydroxy et Z représente l'hydrogène, on réduit un composé de formule I, dans lequel Y et Z représentent ensemble l'hydrogène, avec un complexe de boranate ou de borane-amine,

on convertit les composés de formule I obtenus selon a) à d), dans lesquels R représente l'hydrogène, éventuellement en esters de formule I dans lesquels R représente un groupe alcoyle en $C_1$-$C_6$ ou aralcoyle en $C_7$-$C_9$, et on convertit éventuellement les composés de formule I en leurs sels physiologiquement acceptables.

9. Composé selon l'une quelconque des revendication 1 à 7, destiné à l'emploi comme médicament.

10. Un médicament contenant un composé selon l'une quelconque des revendications 1 à 7.

11. Des composés de formule III

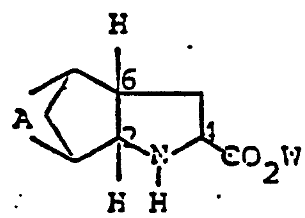

(III)

dans laquelle les atomes d'hydrogène sur les atomes de carbone 2 et 6 sont en configuration cis l'un par rapport à l'autre et le groupe $-CO_2W$ sur l'atome de carbone 4 a une orientation cis ou trans par rapport à l'atome d'hydrogène sur l'atome de carbone 2 et le noyau pyrrolidine a une orientation exo ou endo par rapport au système bicyclique et dans laquelle W représente l'hydrogène ou un groupe éliminable par un acide ou une base ou par hydrogénolyse.

12. Un procédé pour la préparation de composés de formule III selon la revendication 11, selon lequel on transforme des composés de formule XI

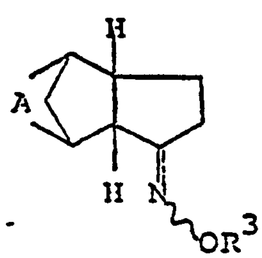

(XI)

dans laquelle A est CH=CH ou $CH_2$-$CH_2$, $R^3$ représente l'hydrogène, un groupe alcoyle, aryle, aralcoyle, $-SO_3H$, benzène sulfonyle, p-toluène sulfonyle, les atomes d'hydrogène sur les atomes de carbone 2 et 6 sont en configuration cis l'un par rapport à l'autre, le noyau cyclopentane une orientation endo ou exo par rapport au système bicyclique, en des composés de formule XII,

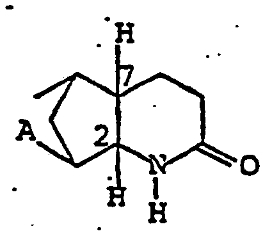

(XII)

dans laquelle A a la signification précédente, les atomes d'hydrogène sur les atomes de carbone 2 et 7 sont en configuration cis et le noyau lactame a une orientation exo ou endo par rapport au système bicyclique, on halogène ceux-ci en composés de formule XIII

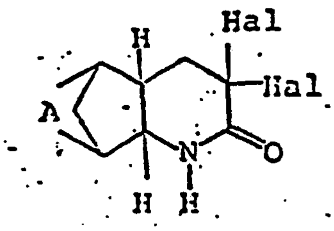

(XIII)

dans laquelle A a la signification ci-dessus et la stéréochimie correspond à la figure XII,
on réduit catalytiquement les composés de formule XIII en composé de formule XIV

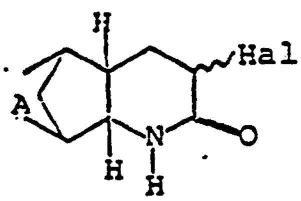

(XIV)

dans laquelle A et Hal ont les significations ci-dessus, et où au cas où A est CH=CH on protège provisoirement cette double liaison sous forme d'un complexe de cyclopentadiénylfer dicarbonyle, on fait réagir les composés de formule XIV obtenus, d'une manière connue en soi en présence d'une base pour obtenir un composé de formule III où W représente l'hydrogène et on estérifie éventuellement ce composé.

13. Composé selon l'une quelconque des rendications 1 à 7, destiné à l'emploi en association avec un diurétique, comme medicament.

14. Un médicament contenant un composé selon l'une quelconque des revendications 1 à 7, en association avec un diurétique,

**Revendications** pour l'Etat Contractant: AT

1. Procédé de préparation de composés répondant à la formule I,

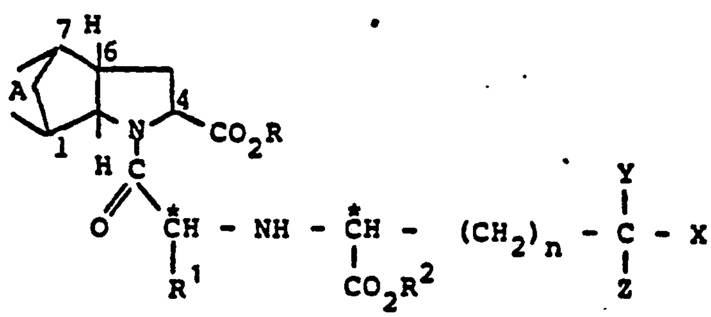

(I)

dans laquelle,
n est 0 ou 1,
A est $-CH=CH-$ ou $-CH_2-CH_2-$,
R représente l'hydrogène, un groupe alcoyle en $C_1$-$C_6$ ou aralcoyle ayant de 7 à 9 atomes de carbone,

$R^1$ représente l'hydrogène ou un groupe alcoyle en $C_1$-$C_6$ qui peut être éventuellement substitué par un groupe amino, acylamino en $C_1$-$C_4$ ou benzoylamino; un groupe alcényle en $C_2$-$C_6$, cycloalcoyle en $C_5$-$C_9$, cycloalcényle en $C_5$-$C_9$, cycloalcoyl ($C_5$-$C_7$)-alcoyle en $C_1$-$C_4$, aryle en $C_6$-$C_{12}$ ou aryle en $C_6$-$C_{12}$ partiellement hydrogéné, qui peuvent être chacun éventuellement substitués par un groupe alcoyle en $C_1$-$C_4$, alcoxy en $C_1$ ou $C_2$ ou par un halogène; un groupe aryl ($C_6$-$C_{12}$) alcoyle en $C_1$-$C_4$ ou aroyl ($C_7$-$C_{13}$)-alcoyle en $C_1$-$C_4$ qui peuvent être tous les deux substitués sur le groupe aryle tel que défini précédemment; un groupe hétérocyclique mono ou bicyclique ayant de 5 à 7 ou de 6 à 10 atomes dans le noyau, dont 1 à 2 atomes dans le noyau représentent des atomes de soufre ou d'oxygène et/ou dont 1 à 4 atomes dans le noyau représentent des atomes d'azote; ou une chaîne latérale d'un acide aminé naturel éventuellement protégé.

$R^2$ représente l'hydrogène, un groupe alcoyle en $C_1$-$C_6$, alcényle en $C_2$-$C_6$ ou aryl ($C_6$-$C_{12}$)-alcoyle en $C_1$-$C_4$,

Y représente l'hydrogène ou un groupe hydroxy,

Z représente l'hydrogène ou

Y et Z représentent ensemble l'oxygène et

X représente un groupe alcoyle en $C_1$-$C_6$, alcényle en $C_2$-$C_6$, cycloalcoyle en $C_5$-$C_9$ aryle en $C_6$-$C_{12}$, qui peut être mono-di- ou tri-substitué par un groupe alcoyle en $C_1$-$C_4$, alcoxy en $C_1$-$C_4$, hydroxy, un halogène, un groupe nitro, amino, alcoylamino en $C_1$-$C_4$, dialcoyl ($C_1$-$C_4$)-amino et/ou méthylène-dioxy; ou un groupe 3-indolyle,

et leurs sels physiologiquement acceptables, caractérisé en ce que:

a) on fait réagir un composé de formule II

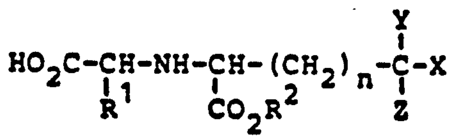

(II)

dans laquelle n, $R^1$, $R^2$, X, Y et Z ont les significations indiquées pour la formule I, avec un composé de formule III

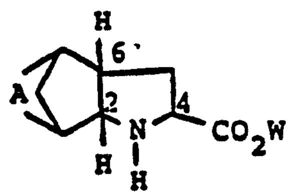

(III)

dans laquelle:

W représente l'hydrogène ou un groupe éliminable par un acide ou une base ou par hydrogénolyse, puis on élimine éventuellement W et/ou $R^2$ en formant les groupes carboxy libres, ou

b) pour la préparation des composés de formule I, dans lesquels Y et Z représentent ensemble l'oxygène,

$b_1$) on fait réagir un composé de formule IV

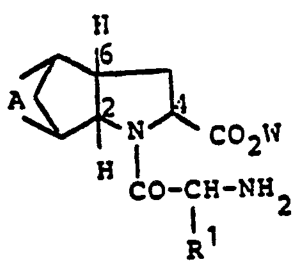

(IV)

dans laquelle $R^1$ a la signification indiquée pour la formule I et W a la signification indiquée pour la formule III, avec un composé de formule V

$$R^2O_2C-CH=CH-CO-X \qquad V$$

dans laquelle $R^2$ et X ont les significations indiquées pour la formule I, et éventuellement on élimine W et/ou $R^2$ en formant les groupes carboxy libres, ou

$b_2$) un composé de formule IV indiqué en $b_1$), avec un composé de formule générale VI, dans lequel $R^2$ a la signification indiquée pour la formule I, et avec un composé de formule générale VII

$OHC-CO_2R^2$ (VI)

$X-CO-CH_3$ (VII)

dans laquelle X a la signification indiquée pour la formule I, et éventuellement on élimine W et/ou $R^2$ en formant les groupes carboxy libres, ou

c) pour préparer des composés de formule I, dans lesquels Y et Z représentent chacun l'hydrogène, on fait réagir un composé de formule IV indiquée en $b_1$) avec un composé de formule VIII

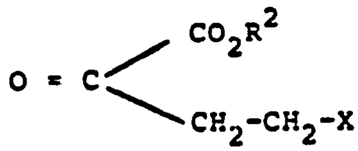

(VIII)

dans laquelle $R^2$ et X ont les significations indiquées pour la formule I, on réduit les bases de Schiff obtenues et éventuellement on élimine W et/ou $R^2$ en formant les groupes carboxy libres, ou

d) pour préparer des composés de formule I, dans lesquels Y représente un groupe hydroxy et Z représente l'hydrogène, on réduit un composé de formule I, dans lequel Y et Z représentent ensemble l'hydrogène, avec un complexe de boranate ou de borane-amine,

on convertit les composés de formule I obtenus selon a) à d), dans lesquels R représente l'hydrogène, éventuellement en esters de formule I dans lesquels R représente un groupe alcoyle en $C_1$-$C_6$ ou aralcoyle en $C_7$-$C_9$, et on convertit éventuellement les composés de formule I en leurs sels physiologiquement acceptables.

2. Procédé selon la revendication 1 pour préparer les composés de formule I dans laquelle les atomes d'hydrogène sur les atomes de carbone, têtes de pont, 2 et 6, sont en configuration cis l'un par rapport à l'autre, le noyau pyrrolidine sur les atomes de carbone 2 et 6 a une orientation endo ou exo par rapport au composé bicyclique et le groupe $-CO_2R$ sur l'atome de carbone 4 a une orientation cis ou trans par rapport à l'atome d'hydrogène sur l'atome de carbone 2.

3. Procédé selon l'une des reveddications 1 ou 2 pour préparer les composés de foxmule I dans laquelle l'atome de carbone en position 4 dans la formule I ainsi que les atomes de carbone marqués d'un astérisque dans la chaîne latérale montrent tous une configuration S.

4. Procédé selon l'une quelconque des revendications 1 à 3 pour préparer les composés de formule I dans laquelle:

n est 1

A est $-CH_2-CH_2-$

R représente l'hydrogène ou un groupe alcoyle en $C_1$-$C_4$,

$R^1$ représente l'hydrogène, un groupe alcoyle en $C_1$-$C_3$, alcényle en $C_2$ ou $C_3$, benzyle, 4-alcoxy-benzyle, phénéthyle, 4-amino-butyle ou benzoylméthyle ou une chaîne latérale d'un acide aminé naturel, éventuellement protégé,

$R^2$ représente l'hydrogène, un groupe alcoyle en $C_1$-$C_4$ ou benzyle,

X représente un groupe phényl qui peut être mono- ou di- substitué par un groupe alcoyle en $C_1$ ou $C_2$, alcoxy en $C_1$ ou $C_2$, hydroxy, le fluor, le chlore, le brome, un groupe amino, alcoylamino en $C_1$-$C_4$, di-alcoyl ($C_1$-$C_4$)-amino, nitro et/ou méthylène dioxy, ou qui peut, dans le cas d'un groupe méthoxy, être trisubsitué par ces mêmes groupes.

5. Procédé selon l'une quelconque des revendications 1 à 4 pour préparer les composés de formule I dans laquelle R représente l'hydrogène, $R^1$ représente un groupe méthyle et X représente un groupe phényle.

6. Procédé selon l'une quelconque des revendications 1 à 5 pour préparer les composés de formule I dans laquelle R représente l'hydrogène, $R^1$ représente un groupe méthyle, X représente un groupe phényle et $R^2$ représente l'hydrogène ou un groupe éthyle.

7. Procédé selon la revendication 2 pour préparer un composé de formule I dans laquelle le groupe carboxy sur l'atome de carbone en position 4 de la formule I a une orientation cis ou trans par rapport à l'atome d'hydrogène sur l'atome de carbone 2, le noyau pyrrolidine a une orientation exo ou endo par rapport au système bicyclique et l'atome de carbone 4 de la formule I ainsi que les atomes de carbone marqués d'un astérisque dans la chaîne latérale ont chacun une configuration S,

n est 1,

A est $-CH_2-CH_2-$,

R représente l'hydrogène,

$R^1$ représente un groupe méthyle,

$R^2$ représente un groupe éthyle,

Y et Z représentent chacun l'hydrogène et

X représente un groupe phényle.

8. Procédé de préparation d'un médicament contenant un composé de formule I selon l'une quelconque des revendications 1 à 7, caractérisé en ce qu'on met ceux-ci sous une forme d'administration appropriée.

9. Procédé de préparation de composés de formule III

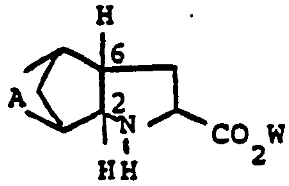

(III)

dans laquelle les atomes d'hydrogène sur les atomes de carbone 2 et 6 sont en configuration cis l'un par rapport à l'autre et le groupe $-CO_2W$ sur l'atome de carbone 4 a une orientation cis ou trans par rapport à l'atome d'hydrogène sur l'atome de carbone 2 et le noyau pyrrolidine a une orientation exo ou endo par rapport au système bicyclique et dans laquelle W représente l'hydrogène ou un groupe éliminable par un acide ou une base ou par hydrogénolyse, caractérise en ce:

qu'on transforme des composés de formule XI

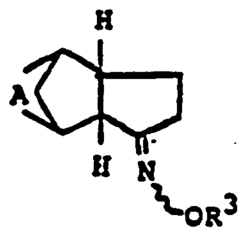

(XI)

dans laquelle A est $CH=CH$ ou $CH_2$-$CH_2$, $R^3$ représente l'hydrogène, un groupe alcoyle, aryle, aralcoyle, $-SO_3H$, benzène sulfonyle, p-toluène sulfonyle, les atomes d'hydrogène sur les atomes de carbone 2 et 6 sont en configuration cis l'un par rapport à l'autre, le noyau cyclopentane a une orientation endo ou exo par rapport au système bicyclique, en des composés de formule XII,

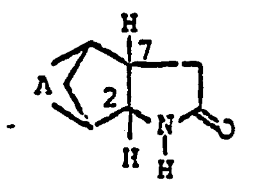

(XII)

dans laquelle A a la signification précédente, les atomes d'hydrogène sur les atomes de carbone 2 et 7 sont en configuration cis et le noyau lactame a une orientation exo ou endo par rapport au système bicyclique, on halogène ceux-ci en composés de formule XIII

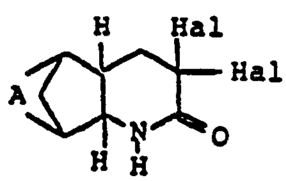

(XIII)

dans laquelle A a la signification ci-dessus et la stéréochimie correspond à la formule XII,

on réduit catalytiquement les composés de formule XIII en composé de formule XIV

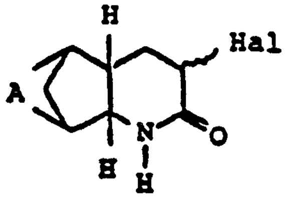

(XIV)

dans laquelle A et Hal ont les significations ci-dessus, et où au cas où A est $CH=CH$ on protège provisoirement cette double liaison sous forme d'un complexe de cyclopentadiényl-fer dicarbonyle, on fait